# EUROPEAN PATENT APPLICATION

(11) **EP 4 276 182 A1**
(43) Date of publication of application: **15.11.2023**
(21) Application number: 22736736.4
(22) Date of filing: 04.01.2022
(51) Int. Cl.: C12N 15/10, A61K 31/40, A61K 31/505, A61K 35/76, A61K 45/00, A61P 31/14, A61P 43/00, C07K 19/00, C12N 5/10, C12N 15/09

(54) **TRANSCRIPTIONAL PRODUCT IN CELLS OF ORGANISM INCLUDING HUMAN, TRANSFECTED RNA, AND TOOL FOR PURIFYING COMPLEX THEREOF**

(30) Priority: 05.01.2021 JP 2021000378
(71) Applicant: Kawasaki Gakuen Educational Foundation, Kurashiki-shi, Okayama 701-0192 (JP)
(72) Inventor: ITO, Tatsuo, Kurashiki-shi, Okayama 701-0192 (JP); SHIMIZU, Yurika, Okayama-shi, Okayama 700-8558 (JP)
(74) Representative: Müller-Boré & Partner Patentanwälte PartG mbB
(86) International application number: PCT/JP2022/000040
(87) International publication number: WO 2022/149568

(57) **Abstract**

The present invention provides a CRISPR-dCas/Cas protein derivative or CRISPR-dCas/Cas protein derivative set derived from a CRISPR-dCas (dead Cas) protein or a CRISPR-Cas protein,
the CRISPR-dCas (dead Cas) protein having a helix region, being capable of forming a complex with a guide RNA and a target RNA, and having no nuclease activity, and
the CRISPR-Cas protein having a helix region, being capable of forming a complex with a guide RNA and a target RNA, and having a nuclease activity,
the CRISPR-dCas/Cas protein derivative or CRISPR-dCas/Cas protein derivative set comprising
an N domain containing the N-terminal side of the helix region of the CRISPR-dCas (dead Cas) protein or the CRISPR-Cas protein,
a C domain containing the C-terminal side of the helix region of the CRISPR-dCas (dead Cas) protein or the CRISPR-Cas protein, and
first and second factors capable of binding in response to stimulation,

the N domain being linked to the first factor, and the C domain being linked to the second factor,
the CRISPR-dCas/Cas protein derivative having the first and second factors being bound via a linker containing a protease recognition sequence or via a linker containing a self-cleaving peptide, or being non-covalently bound,
the CRISPR-dCas/Cas protein derivative thus having
a structure of (N domain)-(first factor)-(linker containing a protease recognition sequence)-(second factor)-(C domain), or
a structure of (N domain)-(first factor)-(linker containing a self-cleaving peptide)-(second factor)-(C domain), or
a structure of (N domain)-(first factor)-(non-covalent bond)-(second factor)-(C domain), and

the CRISPR-dCas/Cas protein derivative set comprising two portions that are (N domain)-(first factor) and (second factor)-(C domain).

## Description

### Technical Field

The present invention relates to a CRISPR-dCas/Cas protein derivative or a CRISPR-dCas/Cas protein derivative set, a polynucleotide or a complementary strand of the polynucleotide, a vector, a transformant, a carrier, a method for purifying a target RNA, a method for analyzing an intracellular environment, a prophylactic or therapeutic agent for a novel coronavirus, and a therapeutic agent for a disease based on abnormal splicing of mRNA.

### Background Art

About 340000 human RNAs are considered to be present in vivo, of which about 300 have been studied, and the functions of about 99% of human RNAs have not been elucidated yet. On the other hand, the number of human proteins is about 21000, of which about 70% have been studied.

The reason why the elucidation of functions of human RNA is delayed is that RNA is a gene that is transiently expressed in each cell, and it is difficult to purify RNA having a specific nucleotide sequence under any conditions because antibody recognition and labeling with, for example, biotin are difficult.

### Summary of Invention

### Technical Problem

An object of the present invention is to provide a targeting technique capable of evaluating a function of an RNA molecule.

Another object of the present invention is to visualize the intracellular behavior of RNA having a specific nucleotide sequence.

Another object of the present invention is to provide a prophylactic or therapeutic agent for a novel coronavirus or a therapeutic agent for a disease based on abnormal splicing of mRNA.

### Solution to Problem

The present invention provides a CRISPR-dCas/Cas protein derivative or a CRISPR-dCas/Cas protein derivative set, a polynucleotide or a complementary strand of the polynucleotide, a vector, a transformant, a carrier, a method for purifying a target RNA, a method for analyzing an intracellular environment, a prophylactic or therapeutic agent for a novel coronavirus, and a therapeutic agent for a disease based on abnormal splicing of mRNA, which are listed below.
Item 1. A CRISPR-dCas/Cas protein derivative or CRISPR-dCas/Cas protein derivative set derived from a CRISPR-dCas (dead Cas) protein or a CRISPR-Cas protein,
   the CRISPR-dCas (dead Cas) protein having a helix region, being capable of forming a complex with a guide RNA and a target RNA, and having no nuclease activity, and
   the CRISPR-Cas protein having a helix region, being capable of forming a complex with a guide RNA and a target RNA, and having a nuclease activity,
   the CRISPR-dCas/Cas protein derivative or CRISPR-dCas/Cas protein derivative set comprising
      an N domain containing the N-terminal side of the helix region of the CRISPR-dCas (dead Cas) protein or the CRISPR-Cas protein,
      a C domain containing the C-terminal side of the helix region of the CRISPR-dCas (dead Cas) protein or the CRISPR-Cas protein, and
      first and second factors capable of binding in response to stimulation,
   the N domain being linked to the first factor, and the C domain being linked to the second factor,
   the CRISPR-dCas/Cas protein derivative having the first and second factors being bound via a linker containing a protease recognition sequence or via a linker containing a self-cleaving peptide, or being non-covalently bound,
   the CRISPR-dCas/Cas protein derivative thus having
      a structure of (N domain)-(first factor)-(linker containing a protease recognition sequence)-(second factor)-(C domain), or
      a structure of (N domain)-(first factor)-(linker containing a self-cleaving peptide)-(second factor)-(C domain), or
      a structure of (N domain)-(first factor)-(non-covalent bond)-(second factor)-(C domain), and
   the CRISPR-dCas/Cas protein derivative set comprising two portions that are (N domain)-(first factor) and (second factor)-(C domain).
Item 2. The CRISPR-dCas/Cas protein derivative or CRISPR-dCas/Cas protein derivative set according to Item 1, wherein the N domain comprises a part of an amino acid sequence on the N-terminal side of the helix region, and the C domain comprises a part of an amino acid sequence on the C-terminal side of the helix region.
Item 3. The CRISPR-dCas/Cas protein derivative or CRISPR-dCas/Cas protein derivative set according to Item 1, wherein the N domain comprises 164 amino acids in a part of an amino acid sequence on the N-terminal side of the helix region of Cas13a derived from *Leptotrichia wadei* (Sequence ID: WP_21746774.1), and the C domain comprises 1003 amino acids in a part of an amino acid sequence on the C-terminal side of the helix region of Cas13a derived from *Leptotrichia wadei* (Sequence ID: WP_21746774.1) .
Item 4. The CRISPR-dCas/Cas protein derivative or CRISPR-dCas/Cas protein derivative set according to any one of Items 1 to 3, wherein the first factor contains nMAG or pMAG and the second factor contains pMAG or nMAG.
Item 5. A polynucleotide encoding the CRISPR-dCas/Cas protein derivative or CRISPR-dCas/Cas protein derivative set of any one of Items 1 to 4, or a complementary strand thereof.
Item 6. One or two vectors comprising one or two polynucleotides of Item 5 or complementary strands thereof.
Item 7. The one or two vectors according to Item 6, wherein each vector is an adenoviral vector, an adeno-associated viral vector, or a plasmid vector.
Item 8. The one or two vectors according to Item 7, wherein the vectors are two vectors, one vector having a structure of (N domain)-(first factor) and the other vector having a structure of (second factor)-(C domain).
Item 9. The one or two vectors according to any one of Items 6 to 8, further comprising a polynucleotide encoding a guide RNA corresponding to the target RNA.
Item 10. A transformant transformed by the one or two vectors of any one of Items 6 to 9.
Item 11. The CRISPR-dCas/Cas protein derivative or the CRISPR-dCas/Cas protein derivative set according to any of Items 1 to 4, the polynucleotide or complementary strand thereof according to Item 5, the one or two vectors according to any one of Items 6 to 9, or the transformant according to Item 10, wherein the target RNA is a group of long noncoding RNAs (lncRNAs) and/or noncoding RNAs (ncRNAs) that are both associated with biological functions and diseases.
Item 12. The transformant according to Item 10 or 11, wherein the transformant is a transformed mammalian cell or a transformed non-human mammal.
Item 13. The CRISPR-dCas/Cas protein derivative or the CRISPR-dCas/Cas protein derivative set according to any one of Items 1 to 4, the polynucleotide or complementary strand thereof according to Item 5, the one or two vectors according to any one of Items 6 to 9, or the transformant according to any one of Items 10 to 12, wherein the target RNA is an RNA derived from an RNA virus, a novel coronavirus RNA, an RNA derived from a synthetic gene, a lncRNA, an ncRNA of 30 nt or more that can be targeted by a guide RNA, or a pre-RNA.
Item 14. A carrier for purifying a target RNA, comprising
   a solid carrier, and
   a CRISPR-dCas (dead Cas) protein capable of forming a complex with a guide RNA and a target RNA and having no nuclease activity, the CRISPR-dCas (dead Cas) protein being supported on the solid carrier.
Item 15. A method for purifying a target RNA, comprising:
   allowing a guide RNA and a target RNA to act on the carrier of claim 14 to form a complex comprising the target RNA, the guide RNA, and the CRISPR-dCas protein on the carrier; and
   eluting the target RNA and a target RNA-binding factor from the complex, the target RNA-binding factor being selected from the group consisting of genomic DNAs, proteins, and RNAs other than the target RNA.
Item 16. A method for analyzing an intracellular environment comprising:
   introducing or expressing multiple guide RNAs into the transformant of Item 10;
   lysing the transformant to capture a target RNA with RNA capture particles; and
   analyzing the target RNA bound to the RNA capture particles.
Item 17. The analysis method according to Item 16, wherein the transformant is an iPS cell.
Item 18. The analysis method according to Item 16 or 17, comprising extracting a phenotype of a cell generated by the formation of a complex of the target RNA, guide RNAs, and a photoactivated dCas protein.
Item 19. A prophylactic or therapeutic agent for a novel coronavirus comprising a lipid metabolism regulator as an active ingredient.
Item 20. The prophylactic or therapeutic agent for a novel coronavirus according to Item 19, wherein the lipid metabolism regulator has an HMG-CoA reductase inhibitory action or an acetyl-CoA reductase inhibitory action.
Item 21. The prophylactic or therapeutic agent for a novel coronavirus according to Item 19 or 20, wherein the lipid metabolism regulator is atorvastatin or rosuvastatin.
Item 22. A therapeutic agent for diseases based on abnormal splicing of mRNA, rRNA (ribosomal RNA), and lncRNA, the therapeutic agent comprising the one or two vectors of any one of Items 6 to 9 as an active ingredient.
Item 23. The CRISPR-dCas/Cas protein derivative or the CRISPR-dCas/Cas protein derivative set according to Item 1, wherein the CRISPR-dCas/Cas protein is Cas13 protein having nuclease activity.

### Advantageous Effects of Invention

According to the present invention, a long non-coding RNA (lncRNA) having a desired nucleotide sequence can be easily purified.

Splitting of Cas 13 protein into peptides based on the splitting position according to the present invention can also be applied to Cas 13 having ribonuclease activity (Cas13) and can be used as activated Cas 13.

The split Cas13/dCas13 according to the present invention can be used for known Cas13/dCas13 applications other than those mentioned above.

According to the present invention, target RNA in a cell can be purified or captured, and the intracellular environment can be analyzed.

The intracellular behavior of lncRNA having the desired nucleotide sequence can be visualized by using labeled stimulusactivated dCas 13.

Further, the present invention can provide a therapeutic agent for a disease mainly caused by abnormal splicing.

Further, the present invention can provide a prophylactic or therapeutic agent for an RNA virus, in particular, a novel coronavirus infection.

### Brief Description of Drawings

Fig. 1 shows a RNA complex purification technique: trans RNA immunoprecipitation (TRIP).
Fig. 2 shows application to modification analysis of RNA function: precision RNA sequence modification (PRSM).
Fig. 3 shows the construction of a photoactivated Cas13 (PAdCas13) system.
Fig. 4 shows the construction of a photoactivated Cas13 (PAdCas13) system.
Fig. 5 shows the construction of a photoactivated Cas13 (PAdCas13) system.
Fig. 6 shows target lncRNAs with high selectivity.
Fig. 7 shows an example of RNA visualization targeting human XIST RNA. The subcellular localization of XIST RNA was able to be confirmed in living cells by using dCas13 labeled with GFP.
Fig. 8 shows a model diagram for implementing the TRIP method.
Fig. 9 is an outline of the TRIP method for the untranslated region of SARS-CoV-2.
Fig. 10 shows a transcript from pGL3-SARS-CoV-2.ORF1-Promoter.
Fig. 11 shows ChIP (chromatin immunoprecipitation) protocol.
Fig. 12 shows performance evaluation 1 of the TRIP analysis method targeting SARS-CoV-2.
Fig. 13 shows performance evaluation 2 of the TRIP analysis method targeting SARS-CoV-2.
Fig. 14 shows kinds of RNA to which RNA derived from SARS-CoV-2 binds in human cells; ontology enrichment analysis.
Fig. 15 shows performance evaluation 3 of the TRIP analysis method targeting SARS-CoV-2.
Fig. 16 shows performance evaluation 4 of the TRIP analysis method targeting SARS-CoV-2.
Fig. 17 is data showing that the hydrogen bond of S565 is involved in the binding of atorvastatin and rosuvastatin to HMG-CoA.
Fig. 18 shows performance evaluation 5 of the TRIP analysis method targeting SARS-CoV-2.
Fig. 19 shows an outline of DNA immunoprecipitation used in the TRIP method.
Fig. 20A shows a demonstration test of a photosensitive switching system.
Fig. 20B shows a performance evaluation test of PA-dCas 13 protein. Western blotting was performed for each cell fraction to confirm the following:
   (A) dCas 13 protein having a guide RNA targeting lncRNA XIST was expressed in the nuclear fraction; and
   (B) dCas 13 was transferred to a site where the RNA targeted by the guide RNA (nucleus) was localized.
Fig. 21 is an outline of an example in which whether the binding region of XIST RNA is related to the histone code was verified.

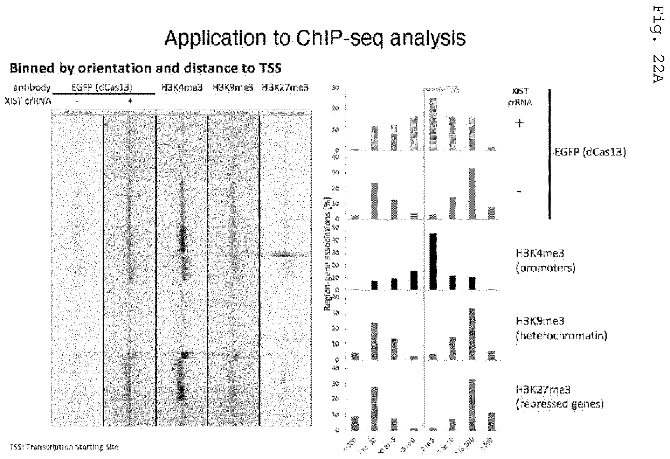

Fig. 22A shows application to ChIP-seq analysis. The diagram on the left in Fig. 22A visualizes whether the binding region on the DNA of XIST RNA has regularity around the transcription start region of the gene. The second column from the left shows test results of the TRIP method for XIST. The first column from the left shows a negative test without crRNA. The third, fourth, and fifth columns from the left are integrated diagrams for histone modification. The diagram on the right is graphs of the accumulation regions from the transcription start region in the diagram on the left. The top graph shows the test result of the TRIP method for XIST, and this result shows an accumulation pattern similar to that of H3K4me3 in the third graph from the top, thus suggesting that XIST is a transcription regulator.

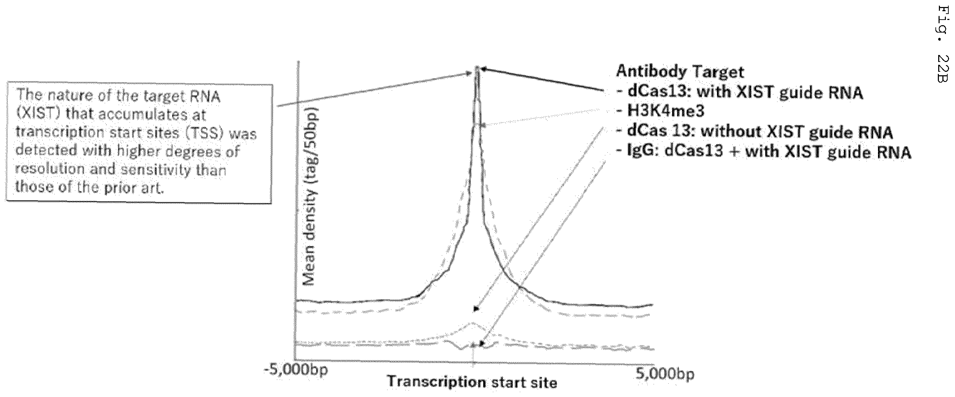

Fig. 22B shows the nature of the target RNA (XIST) that accumulates at the transcription start site (TSS). The target defined by guide RNA was set to a long noncoding RNA XIST, and chromatin immunoprecipitation was performed and compared with the prior art. The technique of the present invention was capable of high-resolution detection with one or two guide RNAs. The ability to turn ON/OFF immediately and the ability to make a comparison within RNA molecules are innovative performance that is difficult to achieve by the prior art and also by the original dCas13 protein. X axis: distance from the transcription start site (the right end: 5000 bp; the left end: -5000 bp); Y-axis: average of accumulation in the transcription start region.
Fig. 23 shows RNA structural analysis data used as a reference in designing XIRT crRNA, a structure prediction diagram, and prediction of a group of proteins that bind to the XIST sequence.
Fig. 24 shows a validation test on the control of XIST RNA function by dCas13. It was confirmed whether the introduction of dCas 13 into a specific sequence of XIST RNA in a cell transfected with a crRNA corresponding to the crRNA No. in the upper diagram of Fig. 23 inhibits the induction of H3 (histone H3) into the genome via XIST RNA. The results show that the binding of H3 to the genome region is inhibited in the cells transfected with crRNAs 1, 2, 3, and 5, and that the function of XIST is inhibited when crRNAs 1, 2, 3, and 5 are targeted.
Fig. 25 shows the application using a photoactivated Cas13 (PAdCas13) system; RNA phenotype analysis using SC-sequence.
Fig. 26 shows Duchenne muscular dystrophy causative factor dystrophin gene mutation repair at the pre-RNA level; development of an exon-skipping therapeutic agent for Duchenne muscular dystrophy.
Fig. 27A shows pLKO5.U6.crRNA (gene name).tRFP.v1.
Fig. 27B shows pLKO5.U6.crRNA (gene name).tRFP.v1.
Fig. 28A shows pENTR1A.dCas13a.GFP.
Fig. 28B shows pENTR1A.dCas13a.GFP.
Fig. 28C shows pENTR1A.dCas13a.GFP.
Fig. 28D shows pENTR1A.dCas13a.GFP.
Fig. 29A shows pGL3-SARS-CoV-2.ORF1-Promoter.
Fig. 29B shows pGL3-SARS-CoV-2.ORF1-Promoter.
Fig. 29C shows pGL3-SARS-CoV-2.ORF1-Promoter.
Fig. 30 shows esiRNA cDNA target sequences which was confirmed to suppress translation via 5' UTR derived from a virus.
Fig. 31 shows PD-1 pre-mRNA of PD-1 positive T cells/NK cells.
Fig. 32 shows guide RNAs targeting introns 1 and 2 of PD -1 pre-mRNA.
Fig. 33 shows preparing PD-1-inactivated lymphocytes using dCas13. PD-1 expression-regulated T cells were successfully produced by using comprehensive RNA targeting analysis. The development of an ex vivo pharmaceutical can be made by using the guide RNA sequence and dCas13 protein contained in the cells in the area a shown in the diagram on the right in Fig. 33: a cell therapy drug that is safer than CAR-T cell therapy (Kymriah) can be provided.
Fig. 34 shows that increased translation activity was observed in luciferase RNA fused with SARS-CoV-2-derived 5' UTR RNA gene information.

### Description of Embodiments

Examples of the target RNA for isolation and purification, functional elucidation, and disease prevention or treatment in the present invention include lncRNA, RNAs derived from RNA viruses, including novel coronavirus RNA, and RNAs having abnormal splicing.

In the present invention, examples of the CRISPR-dCas protein having a helix region, being capable of forming a complex with a guide RNA and a target RNA, and having no nuclease activity or the CRISPR-Cas protein having a helix region, being capable of forming a complex with a guide RNA and a target RNA, and having a nuclease activity include Cas5/dCas5, Cas6/dCas6, Cas7/dCas7, Cas9/dCas9, Cas10/dCas10, Cas11/dCas11, and Cas13/dCas 13, among which Cas13/dCas 13 is preferred.

The CRISPR-dCas protein and the CRISPR-Cas protein have a helix region, are cleaved at any position in the helix region, and are divided into an N domain on the N-terminal side of the cleavage point and a C domain on the C-terminal side of the cleavage point to form the following two polypeptides: a polypeptide of (N domain)-(first factor) in which the N domain and the first factor are bound, a polypeptide of (second factor)-(C domain) in which the C domain and the second factor are bound. By binding these polypeptides via a linker comprising a protease recognition sequence or a linker comprising a self-cleaving peptide, one protein (CRISPR-dCas/Cas protein) represented by (N domain)-(first factor)-(linker containing a protease recognition sequence)-(second factor)-(C domain) or (N domain)-(first factor)-(linker containing a self-cleaving peptide)-(second factor)-(C domain) may be formed. Alternatively, a polypeptide comprising (N domain)-(first factor) and a polypeptide comprising (second factor)-(C domain) may be used as a set (a CRISPR-dCas/Cas protein derivative set). This set is "the CRISPR-dCas/Cas protein derivative set comprising two portions." One protein represented by (N domain)-(first factor)-(linker comprising a protease recognition sequence)-(second factor)-(C domain) or one protein represented by (N domain)-(first factor)-(linker comprising a self-cleaving peptide)-(second factor)-(C domain) is cleaved with a protease recognition sequence or a self-cleaving peptide to form a set of polypeptides, one peptide comprising (N domain)-(first factor) and the other polypeptide comprising (second factor)-(C domain). When the first factor and the second factor are bound by stimulation, one complex represented by (N domain)-(first factor)-(non-covalent bond)-(second factor)-(C domain) is formed. This complex and proteins represented by (N domain)-(first factor)-(linker comprising protease recognition sequence)-(second factor)-(C domain) or represented by (N domain)-(first factor)-(linker comprising self-cleaving peptide)-(second factor)-(C domain) are sometimes collectively referred to as the "CRISPR-dCas/Cas protein derivative" in the present specification. When a non-covalent bond in the complex is broken in the absence of stimulation, the complex splits into a set of polypeptides, one peptide comprising (N domain)-(first factor) and the other polypeptide comprising (second factor)-(C domain). The formation and breaking of the non-covalent bond between the first factor and the second factor reversibly occur in the presence or absence of stimulation. Examples of the stimulation that promotes the binding of the first factor and the second factor include physical stimuli, such as light (e.g., white light and blue light), heat (high temperature, low temperature), pH (acid, neutral, alkaline), and pressure; a combination of a ligand and a receptor; and chemical substances. When the stimulation is light, either the first factor or the second factor may be nMAG, and the other may be pMAG. The "non-covalent binding" includes binding of the first factor and the second factor. For example, when the first factor and the second factor are nMAG and pMAG, the "non-covalent bond" includes the binding of nMAG and pMAG.

The protease recognition sequence may be cleaved by an intracellular protease. A protease capable of cleaving the protease recognition sequence may be introduced into cells together with the CRISPR-dCas/Cas protein. The length of the linker may be appropriately selected by a person skilled in the art, but is preferably a peptide comprising 3 to 100 amino acids, and more preferably 5 to 60 amino acids. As the protease recognition sequence, various protease recognition sequences known in the art can be used (J. Clin. Biol. Chem., Vol. 283, No. 30, p. 20897, 2008). Preferably, the protease recognition sequence is an intracellular protease recognition sequence in the cell into which a CRISPR-dCas/Cas protein derivative or CRISPR-dCas/Cas protein derivative set is introduced. Examples of the intracellular protease recognition sequence include amino acid sequences recognized by the intracellular proteases Furin, PC7, and PACE4. The linker may be a peptide consisting of a protease recognition sequence or may further contain an amino acid sequence on the N-terminal side and/or the C-terminal side of the protease recognition sequence. Examples of self-cleaving peptides include those described in the literature (Szymczak-Workman, Andrea L et al., "Design and construction of 2A peptide-linked multicistronic vectors." Cold Spring Harbor Protocols, vol. 2012, 2 199-204. 1 Feb. 2012, doi: 10.1101/pdb. ip 067876.), which is incorporated herein by reference in its entirety. Examples of the self-cleaving peptide include, but are not limited to, T2A, P2A, E2A, and F2A.

The N domain and the C domain may or may not have a part of the amino acids in the helix region, may have a modified amino acid sequence in the helix region, may comprise a new amino acid sequence added or inserted to the original amino acid sequence, or may comprise an amino acid sequence derived from a protease recognition sequence or a self-cleaving peptide or a linker.

The present invention includes a polynucleotide encoding the CRISPR-dCas/Cas protein derivative, or the CRISPR-dCas/Cas protein derivative set, or a complementary strand of the polynucleotide. Examples of such polynucleotides include:
(i) a polynucleotide encoding (N domain)-(first factor)-(linker comprising a protease recognition sequence)-(second factor)-(C domain) or (N domain)-(first factor)-(linker comprising a self-cleaving peptide)-(second factor)-(C domain);
(ii) one polynucleotide formed by linking a polynucleotide encoding a polypeptide comprising (N domain)-(first factor) and a polynucleotide encoding a polypeptide comprising (second factor)-(C domain) via an appropriate spacer nucleotide sequence; and
(iii) a set of two polynucleotides, one polynucleotide encoding a polypeptide comprising (N domain)-(first factor) and the other polynucleotide encoding a polypeptide comprising (second factor)-(C domain).

The vector of the present invention includes any one of the above (i) to (iii). In the case of (i) and (ii), the vector is one vector. In the case of (iii), the vector is a set of two vectors.

The vector may be a plasmid vector or a viral vector. Examples of viral vectors include adeno-associated viral vectors, adenoviral vectors, retroviral vectors, and lentiviral vectors. Examples of plasmid vectors include an entry vector for the Gateway system (e.g., pENTR), a donor vector for gene recombination, and a destination vector for parallel transfer.

The vector may encode at least one guide RNA in addition to the polynucleotide encoding the CRISPR-dCas/Cas protein derivative or the CRISPR-dCas/Cas protein derivative set, or a complementary strand of the polynucleotide.

The transformant of the present invention is produced by transforming a host cell with a vector of the present invention. Examples of the host include mammals, such as humans, mice, rats, rabbits, guinea pigs, hamsters, goats, dogs, and monkeys. When the host cell is an ES cell or a fertilized egg of a mammal other than a human, a non-human mammal can be produced from the transformed cell. The transformant of the present invention includes non-human mammals.

The carrier for purifying a target RNA of the present invention comprises a solid carrier on which multiple CRISPR-dCas (dead Cas) proteins capable of forming a complex with a guide RNA and a target RNA and having no nuclease activity are supported on the solid carrier. Examples of the CRISPR-dCas (dead Cas) protein having no nuclease activity include dCas5, dCas6, dCas7, dCas9, dCas10, dCas11, and dCas13, among which dCas13 is preferred.

Examples of solid carriers for supporting the CRISPR-dCas protein include known carriers, such as diatomaceous earth, activated carbon, alumina, titanium oxide, crosslinked starch particles, cellulose-based polymers, chitin, and chitosan derivatives.

Examples of the method for immobilizing the CRISPR-dCas protein on a solid carrier include methods known as methods for immobilizing a protein, such as the physical adsorption method, the ion binding method, the comprehensive method, and the covalent binding method. Among these methods, the covalent binding method is preferable because of its excellent long-term stability. Examples of the method for covalently bonding the dCas protein include various methods such as a method using a compound having an aldehyde group, such as formaldehyde, glyoxal, or glutaraldehyde; a method using a multifunctional acylating agent; and a method of crosslinking a sulfhydryl group. The carrier for purifying target RNA is preferably used by filling a column reactor.

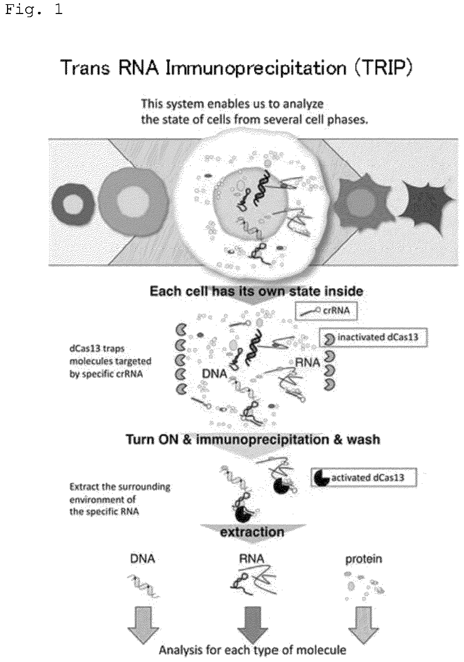

Fig. 1 illustrates a method for functional analysis of target RNA in cells or a cell lysate using the CRISPR-dCas/Cas protein derivative of the present invention. In Fig. 1, photoactivated dCas13 is used as the CRISPR-dCas protein, inactivated dCas13 is dCas13 before irradiation with blue light or white light (dark place), and activated dCas13 is dCas13 after irradiation with light, including blue light or white light (light place). Before the light irradiation, crRNA, inactivated dCas13, and target RNA are present separately. When the activated dCas13 is produced by light irradiation, a crRNA-activated dCas 13-target RNA complex is formed. The target RNA may be bound to DNA, RNA other than the target RNA, protein, or the like.

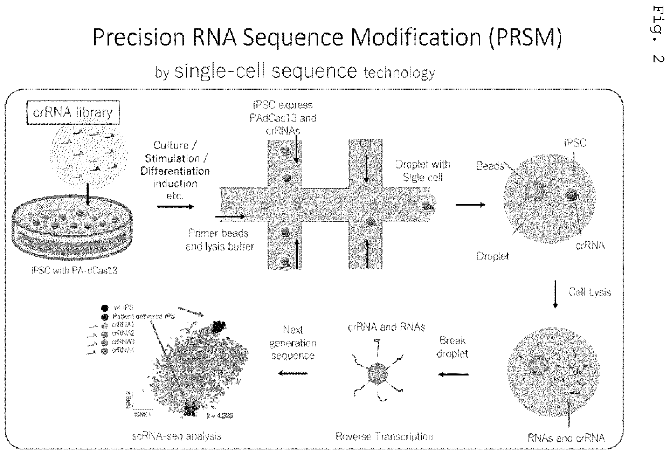

Fig. 1 shows an example in which DNA, RNA, or a protein is complexed with the target RNA. When DNA or RNA is bound to the target RNA, the DNA or RNA can be separated from the target RNA and analyzed by real-time PCR (qPCR) next-generation sequencing (NGS) to identify the DNA or RNA to which the target RNA is bound. When a protein is bound to the target RNA, the protein bound to the target RNA can be specified by separating the protein from the target RNA and performing proteome analysis. The function of the target RNA can be thus clarified by analyzing the DNA, RNA, or protein to which the target RNA is bound. Further, the sequence of the target RNA can be determined by analysis using a combination of qPCR and NGS. These analyses can be used to analyze the intracellular environment. For example, Fig. 2 shows the results of analysis of the intracellular environment after differentiation induction of iPS cells.

The crRNA sequence can be designed based on the crRNA algorithm based on RNA higher-order structure prediction. A carrier for purifying a target RNA is designed to comprise many crRNAs bound to the CRISPR-dCas protein. A large number of target RNAs in the sample that are bound to the crRNAs are sequenced, whereby information on the target RNAs can be obtained. For example, a group of long noncoding RNAs (lncRNAs) expressed during a disease can be determined from samples derived from cells of the disease, or if the sequence of a group of lncRNAs expressed during a disease has already been determined, an effective drug candidate compound can be screened by monitoring how the expression pattern is changed by the drug candidate compound. Since many relationships between specific diseases and lncRNAs are known, crRNAs can be designed with reference to such information.

In one preferred embodiment of the present invention, a photoactivated dCas protein system (PAdCas system) may be used to observe the expression, distribution, migration, etc. of lncRNA in cells.

Fig. 2 shows an example in which the cell of the present invention is applied to PRSM (precision RNA sequence modification).

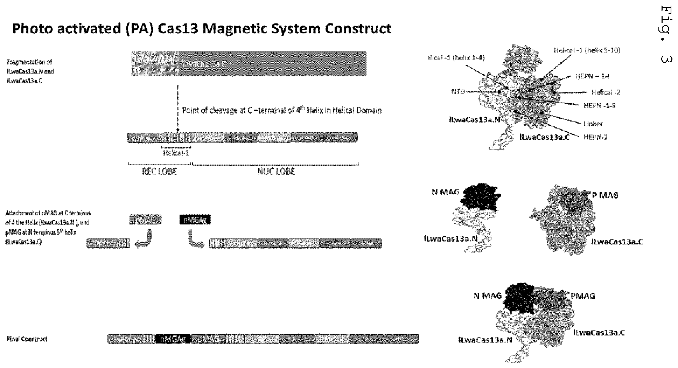

Figs. 3 to 5 show an example of the PAdCas system of the present invention. In the PAdCas system, the CRISPR-dCas protein is divided into an N-terminal portion comprising a guide RNA recognition domain (corresponding to ILwaCas13a.N-N MAG in Figs. 3 to 5) and a second portion comprising a HEPN domain (corresponding to ILwaCas13a.C-P MAG in Figs. 3 to 5)in a dark place. When a cell comprising these portions is irradiated with blue light or white light, the conformation of nMAG and pMAG is changed, and the nMAG and pMAG bind to each other to become an active dCas protein, and a complex formed by binding of a crRNA and a target RNA in a cell can be detected by the labeling of active dCas (for example, a fluorescent protein, such as a green fluorescent protein). The nucleotide sequences and amino acid sequences of nMAG and pMAG are known in the literature (Nature Communications, vol. 6, Article No.: 6256 (2015)).

The CRISPR-Cas/dCas protein used in the present invention, which is capable of forming a complex with a guide RNA and a target RNA and has or does not have RNA nuclease activity, is capable of regulating the RNA splicing mechanism. For example, in the case of a Duchenne muscular dystrophy patient with a stop codon introduced into the 44th exon, the guide RNA for skipping Cas13 and exon 44 of the present invention can be introduced into cells to treat Duchenne muscular dystrophy.

Diseases mainly caused by abnormal RNA splicing, such as Duchenne muscular dystrophy, are shown in Tables 1 to 4 below.

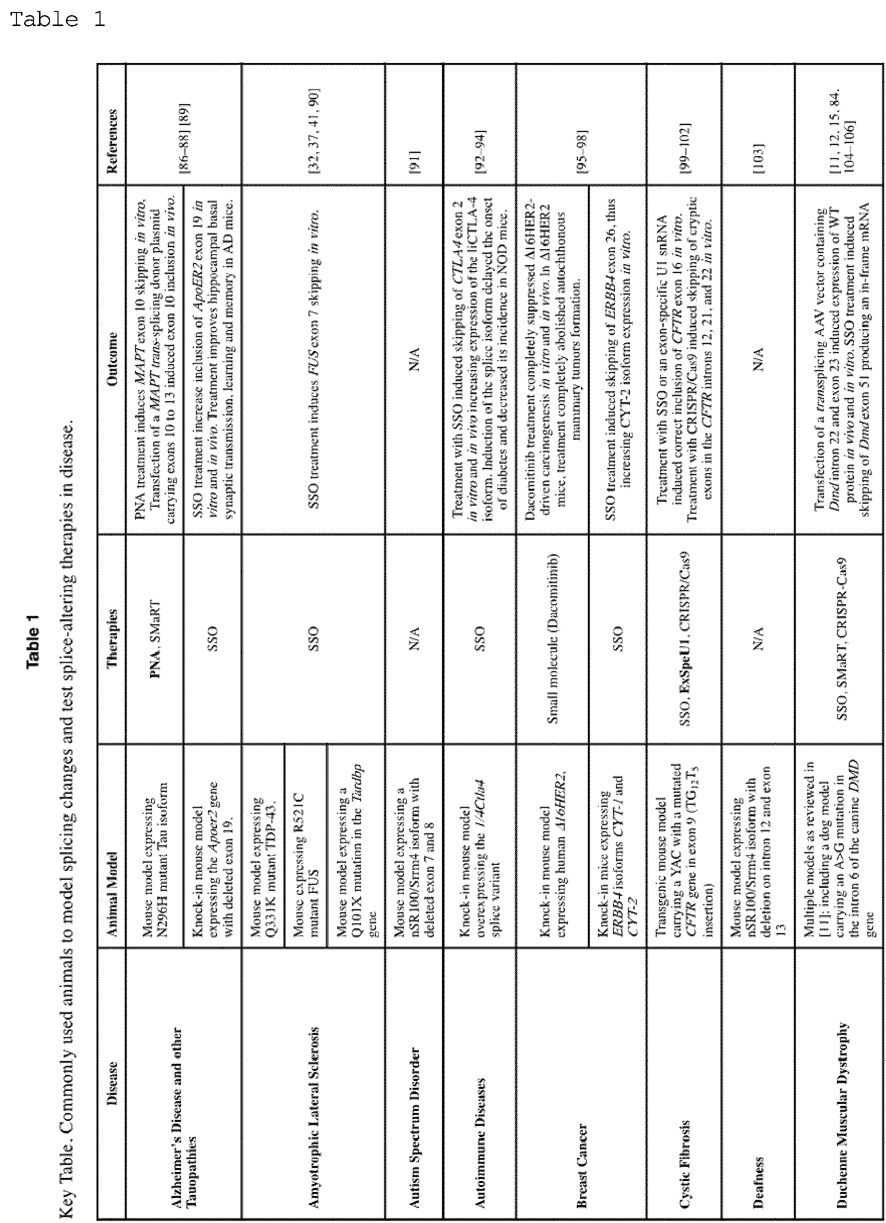

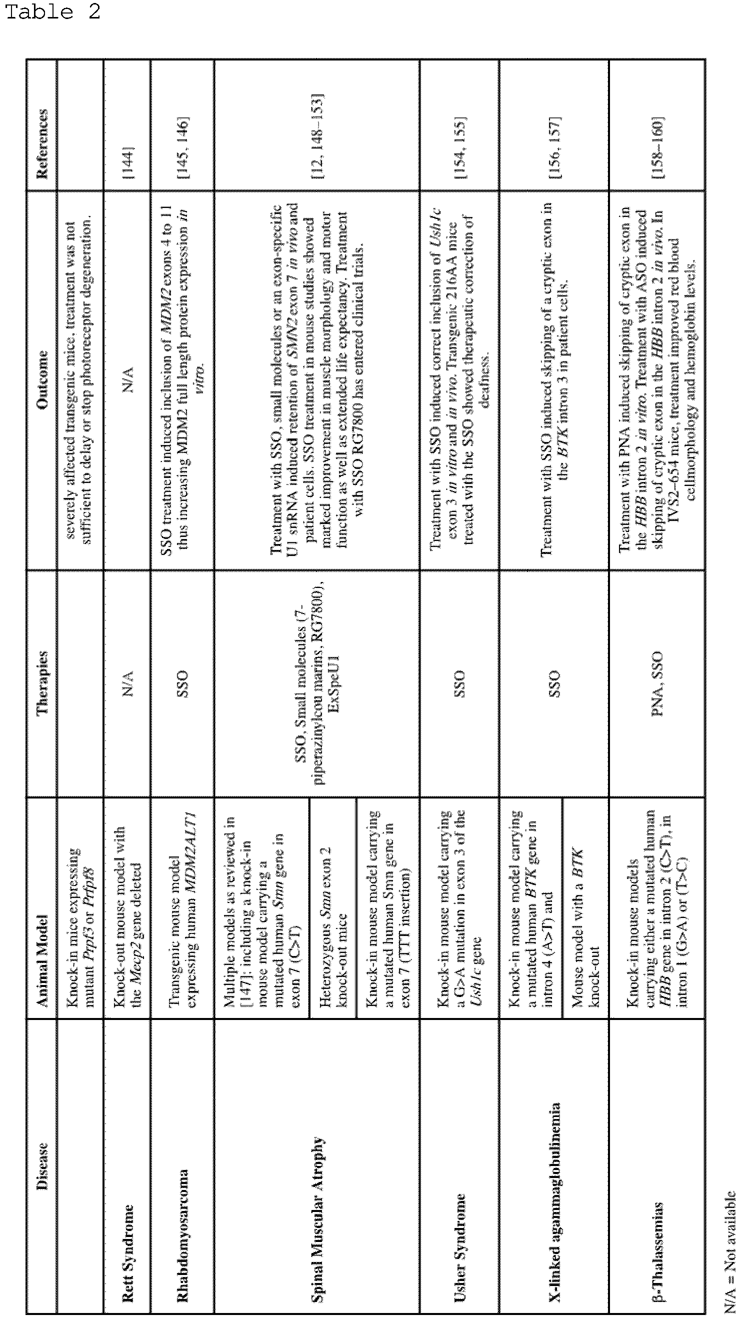

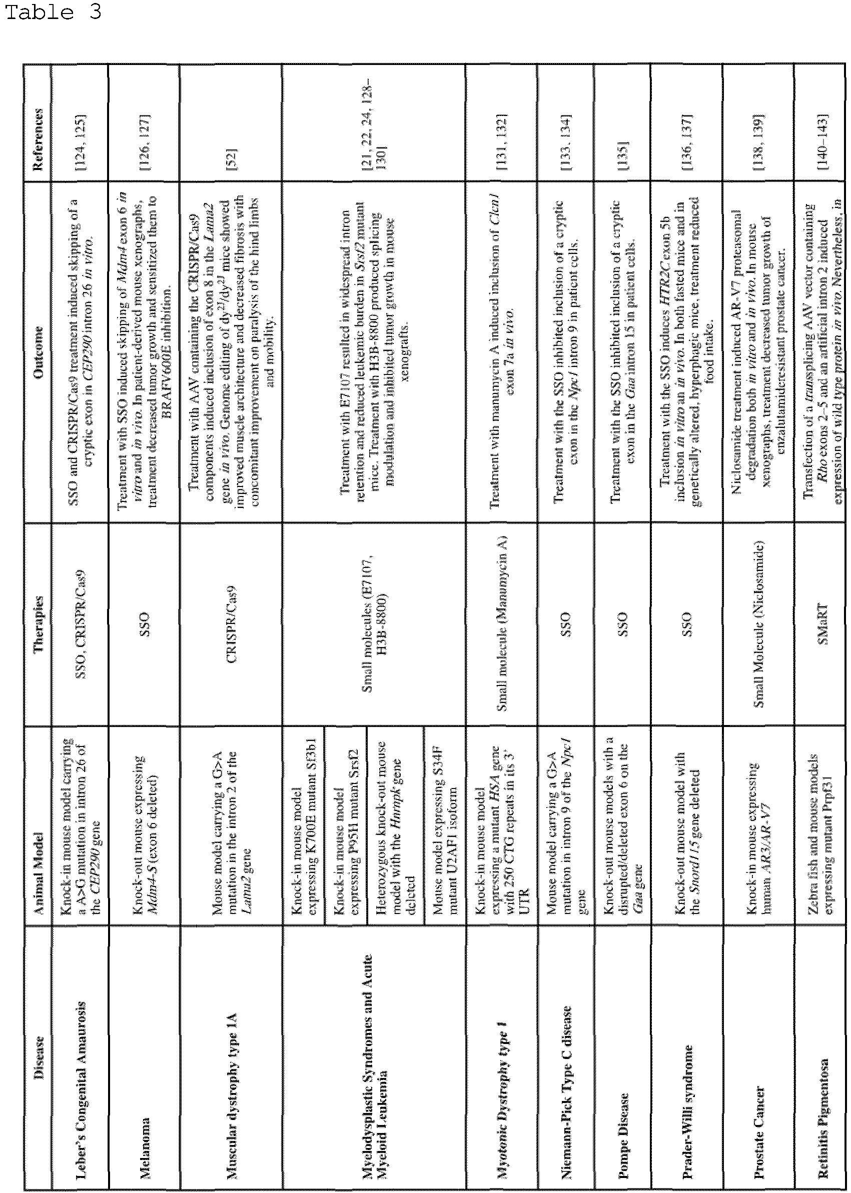

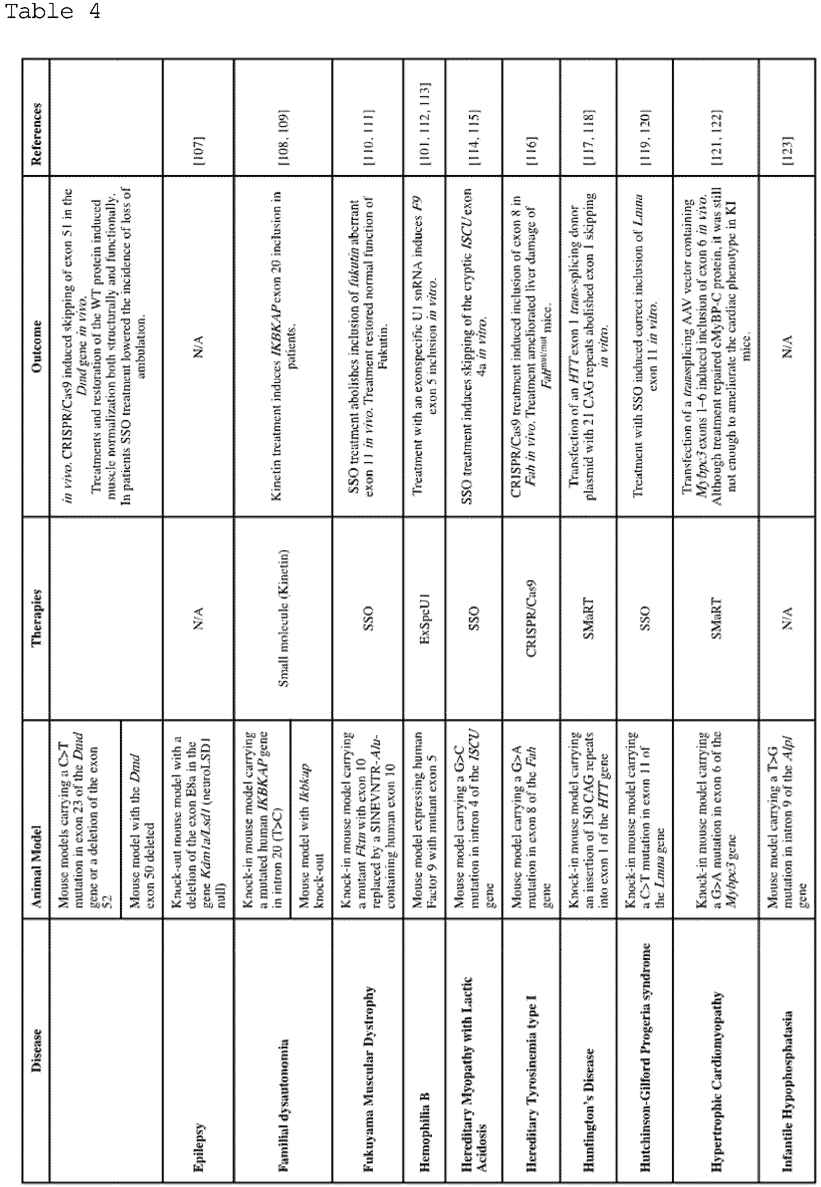

The present invention further provides a therapeutic agent for a novel coronavirus capable of suppressing the growth of the novel coronavirus in the body of a mammal, including a human.

The research of the present inventors has revealed that the proteins of novel coronaviruses are biosynthesized using lipid metabolic pathways of mammals, particularly humans. The present inventors tested a lipid metabolism regulator as a substance capable of suppressing the biosynthesis of a novel coronavirus protein in a lipid metabolic pathway, thereby preventing viral infection, and further suppressing the growth of a virus even when the viral infection is established, thereby alleviating the symptoms of a novel coronavirus infection and suppressing the severity. As a result, the present inventors found that the lipid metabolism regulator can prevent the infection of a novel coronavirus by administering the lipid metabolism regulator to mammals, such as humans, especially to humans who have not been given lipid metabolism regulators; even in the case of a novel coronavirus infection, the lipid metabolism regulator can suppress or at least delay the transition from mild to moderate disease or from moderate to severe disease. The lipid metabolism regulator, when taken before infection with a novel coronavirus, can inhibit the growth of the novel coronavirus in the body, thereby inhibiting the onset of infection. The lipid metabolism regulator is particularly useful in inhibiting novel coronavirus infection because of its low toxicity. The dosage of the lipid metabolism regulator is about 1 to 150 mg per day for an adult human. The lipid metabolism regulator can be administered in divided doses 1 to 4 times per day.

Examples of the lipid metabolism regulator include, but are not limited to, mevastatin, atorvastatin, pravastatin, rosuvastatin, fluvastatin, and lovastatin. The lipid metabolism regulator preferably has an HMG-CoA reductase inhibitory action. Preferred examples of lipid metabolism regulators include atorvastatin and rosuvastatin.

PD-1 can be suppressed by applying the present invention to NK cells. For example, pleural dissemination or peritoneal dissemination can be treated by removing pleural effusion or ascitic fluid from a patient with pleural dissemination or peritoneal dissemination, suppressing PD-1 of NK cells contained in the pleural effusion or ascitic fluid, and returning the resulting cells to the pleural effusion or ascitic fluid.

For example, a combination of anti-PD-1 antibody with the dCas 13 complex of the present invention can be used.

Inhibition of normal PD-1 mRNA partially maturation by using anti-PD1 antibody and the dCas13 complex of the present invention can more effectively treat cancer by PD-1-expression-inhibiting T cells, which is eliminated self tolerance while maintaining the original T cell activity. Specifically, dCas13, which targets PD-1 pre-mRNA, can be expressed in human lymphocyte cells for splicing regulation, depending on external stimulation.

### Examples

Embodiments of the present invention are described below in detail based on Examples.

### Example 1: RNA Complex Purification Procedure Using Trans RNA Immunoprecipitation (TRIP) (Fig. 1)

The RNA target sequence recognition and binding system known as a CRISPR-dCas protein (e.g., CRISPR-dCas 13; hereafter sometimes abbreviated as "dCas13") is a system in which a complex of a guide RNA (crRNA) comprising 28 nucleotides of a target sequence and a dCas protein having no nuclease activity and designed to be divided into two portions, such as a combination of pMAG and nMag capable of binding by light irradiation, recognizes a target gene and forms a complex with the target gene. For example, in Fig. 1, in the case of dCas 13 divided into pMAG and nMag, inactivated dCas 13 corresponds to a state in which pMAG and nMAG are separated before light irradiation, and activated dCas 13 corresponds to a state in which pMAG and nMAG are bound after light irradiation. The inactivated dCas13 and activated dCas13 can be reversibly changed between the bound and unbound states depending on, for example, the temperature at which a heat shock protein system is used. The divided dCas protein is in an inactive state, but can be bound and activated by external stimulation, such as light or heat. Here, "activated" means that the CRISPR-dCas protein forms a complex of the target RNA and crRNA. The use of TRIP technology allows for extraction of a complex of the target RNA, crRNA, and dCas13. The complex can include other RNAs, DNAs, proteins, and like various factors that bind to the target RNA. In some cases, it is also possible to eliminate specific RNA (such as ribosomal RNA) from the cell extract. In Fig. 1, "Turn ON & immunoprecipitation & wash" means that inactivated dCas13 is converted into activated dCas13 by light irradiation (Turn ON) and the obtained complex is immunoprecipitated and then washed to remove components other than the complex. Subsequent extraction separates the target RNA from DNA, protein, or non-target RNA bound to the target RNA, and the DNA/RNA can be sequenced by next generation sequencing. In the case of proteins, proteome analysis can be performed.

Fig. 1 shows the application of TRIP technology to a cell extract. However, in TRIP technology, the RNA labeling method using Cas13 or dCas13 can be applied intracellularly or to cell extracts.

### Example 2

### (1) Application to RNA Function Modification Analysis (Precision RNA Sequence Modification (PRSM))

Target RNA labeling with Cas13 or dCas13 itself modifies the function of the targeted RNA. Taking advantage of this fact enables comprehensive analysis of RNA function in each cell transfected with crRNA library. The functional modification of the target RNA can be adjusted by switching from Inactive-Cas13/dCas13 to Active-Cas13/dCas13 by external stimulation, thus enabling the observation of phenomena in the cell transfected with crRNA and either Cas13 or dCas13 at the time desired by the tester.

Fig. 2 illustrates an application in droplet sequencing (Drop-Seq). When droplet sequencing is performed at a time desired by the tester on a cell group in which any crRNA (crRNA library in Fig. 2) and Cas13/dCas13 have been introduced and inactive-Cas 13/dCas 13 has been switched to active-Cas 13/dCas 13, the function of RNA targeted by the crRNA is modified by active-Cas13/dCas13. As a result, since each cell exhibits a different phenotype that varies depending on the sequence of the introduced crRNA, next-generation sequencing enables analysis of the expression level of a transcript as a phenotype of each cell. Since single-cell RNA-seq (scRNA-seq) technology can be used to track the trajectories of various cell lineages, the use of crRNA libraries pre-designed at the molecular and cell biological level enables the identification of the phenotype of target RNA and the sequences responsible for RNA function. A large number of sequences complementary to various RNAs (e.g., antisense polynucleotides of a target RNA and a guide RNA) are bound to beads in Fig. 2, and the target RNA binds to the beads.

Droplet sequencing is a known technique (references: Shapiro E et al. Single-cell sequencing-based technologies will revolutionize whole-organism science. Nat Rev Genet. 2013 Sep. 14(9): 618-30. doi: 10.1038/nrg3542. Epub 2013 Jul 30. PMID: 23897237; Alizadeh AA et al. Toward understanding and exploiting tumor heterogeneity. Nat Med. 2015 Aug. 21(8): 846-53. doi: 10.1038/nm.3915. PMID: 26248267. PMCID: PMC 4785013). Fig. 2 shows an embodiment in which droplet sequencing is combined with the present invention, in which crRNA and photoactivated dCas13 (PA-dCas13) are introduced into iPS cells (iPSCs). PA-dCas13 and crRNA may be introduced into iPS cells by a known method capable of introducing proteins and RNA into cells such as liposomes and lipofectamine; or a plasmid vector, a viral vector, or the like encoding PA-dCas 13 and crRNA may be introduced into iPS cells to express the crRNA and photoactivated dCas13 (PA-dCas13) in the cells.

### (2) Construction of Photoactivated Cas13 (PAdCas13) System

The structure of Cas13 protein was analyzed and divided into two peptides in the Helical 1 region, which is expected to have little effect on recognition of target RNAs, such as HEPN-1 and HEPN-2, and on binding to crRNA (Fig. 3). Each of the divided peptides was split into two peptides as an N-terminal peptide of dCas13 (Cas13a.N:REC LOBE) and a C-terminal peptide (Cas13a.C:NUC LOBE) of dCas13.

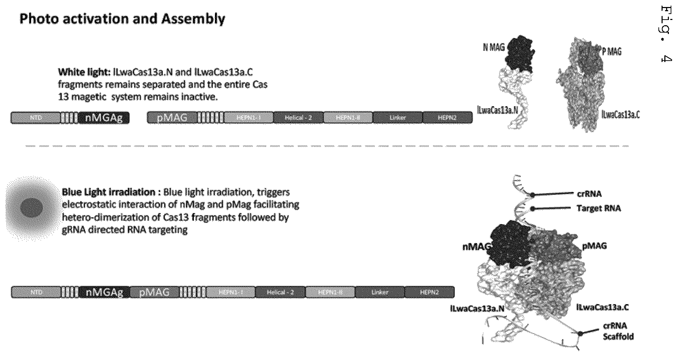

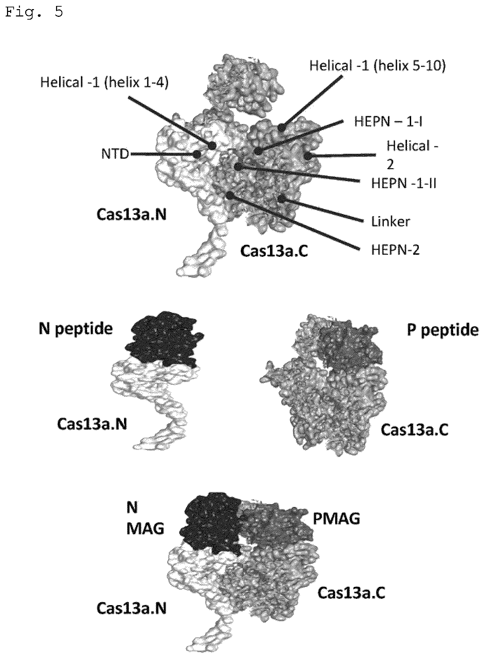

In this Example, peptides (pMAG and nMAG) having a property of binding photosensitively were added to the C-terminal side of dCas13a.N and the N-terminal side of dCas13a.C, respectively, whereby a mechanism was created in which the two divided peptides were recombined by light (blue light) stimulation, thus enabling the resulting Cas13 protein to reacquire its original function of forming a complex with a target RNA under the induction of crRNA (Figs. 4 and 5). Methods that can be used to reacquire the function by the divided Cas13/dCas13 peptides include not only physical stimuli, such as light stimulation, temperature, and pressure, but also alternative chemical stimuli, such as ion gradients and drug stimulation.

### Example 3: RNA Purification Test with crRNA Targeting Human XIST RNA

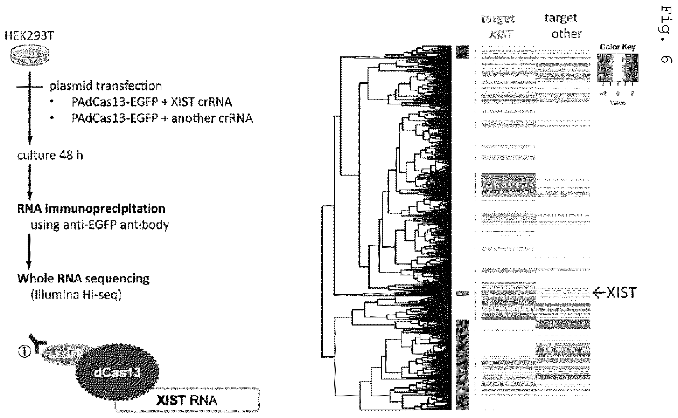

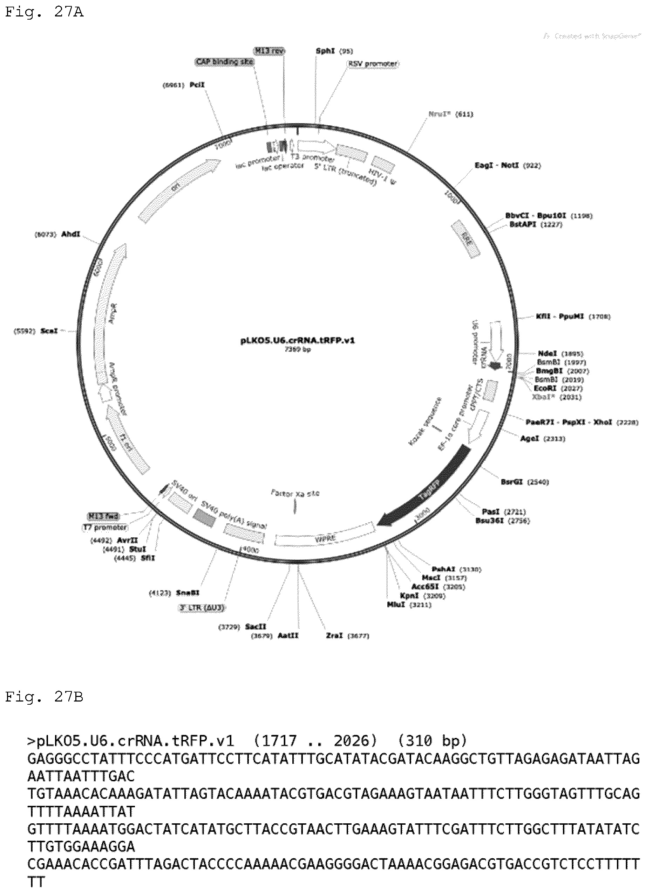

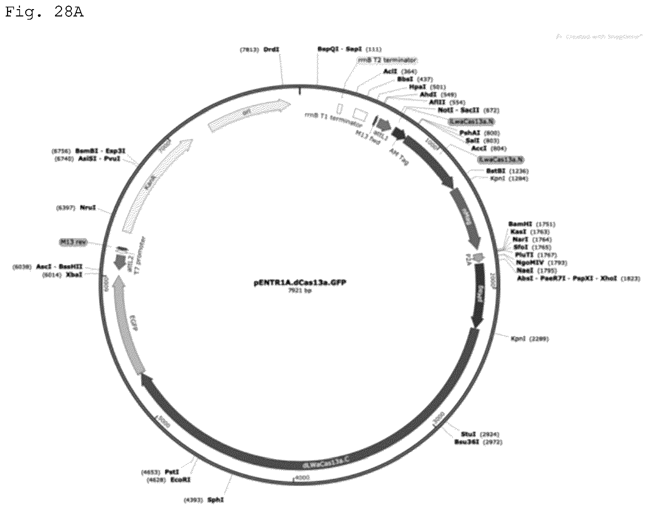

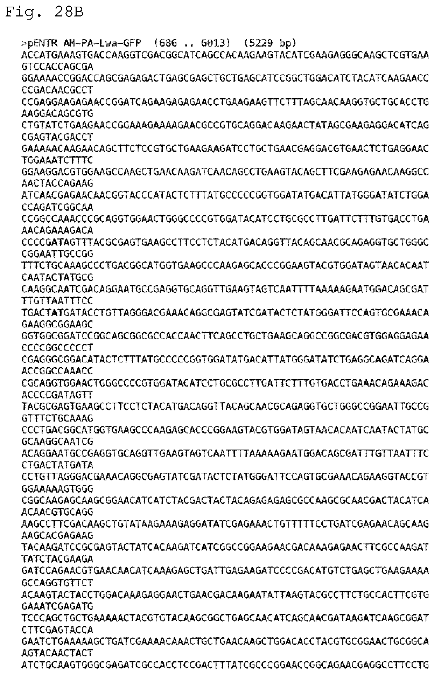

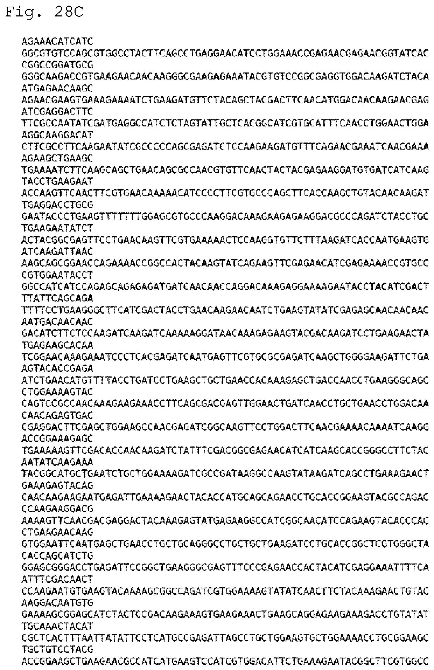

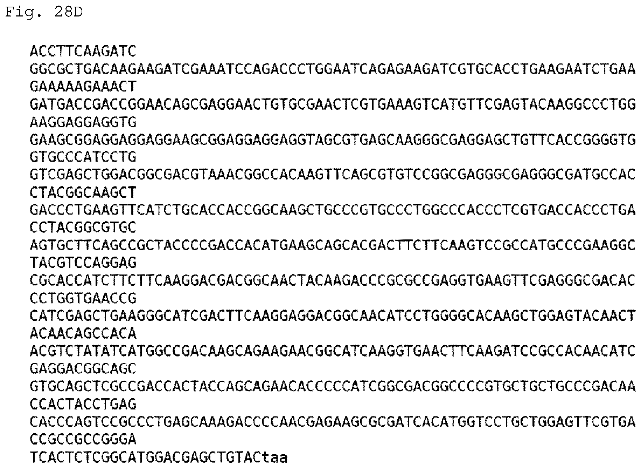

Using the RNA ChIP method, XIST RNA and RNA binding to XIST were purified from a cell extract by immunoprecipitation via dCas13 (TRIP method). The results confirmed that that XIST RNA can be extracted with a high degree of purity by the next-generation sequencing method (Fig. 6). pENTR1A.dCas13a.GFP (Fig. 28A) was created as an entry vector for Gateway (registered trademark) specific recombination and recombined into a mammalian cell expression vector. A lentiviral vector constitutively expressing a crRNA targeting XIST was created by inserting the XIST gene sequence into pLKO5.U6.crRNA(gene name).tRFP.v1 (Fig. 27A) .

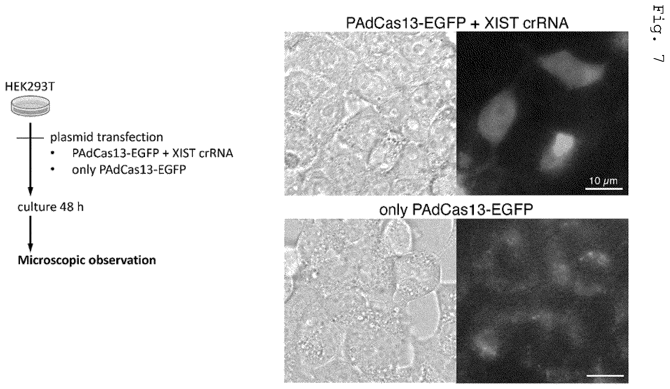

Fig. 7 shows an example of RNA visualization targeting human XIST RNA. By using photoactivated dCas13 (PAdCas13) labeled with GFP, the nuclear localization of XIST RNA could be confirmed in living cells.

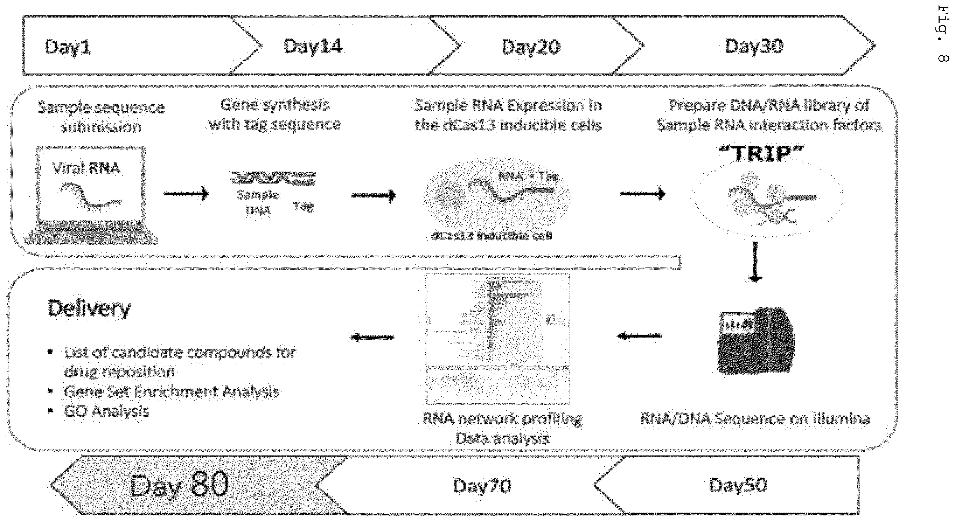

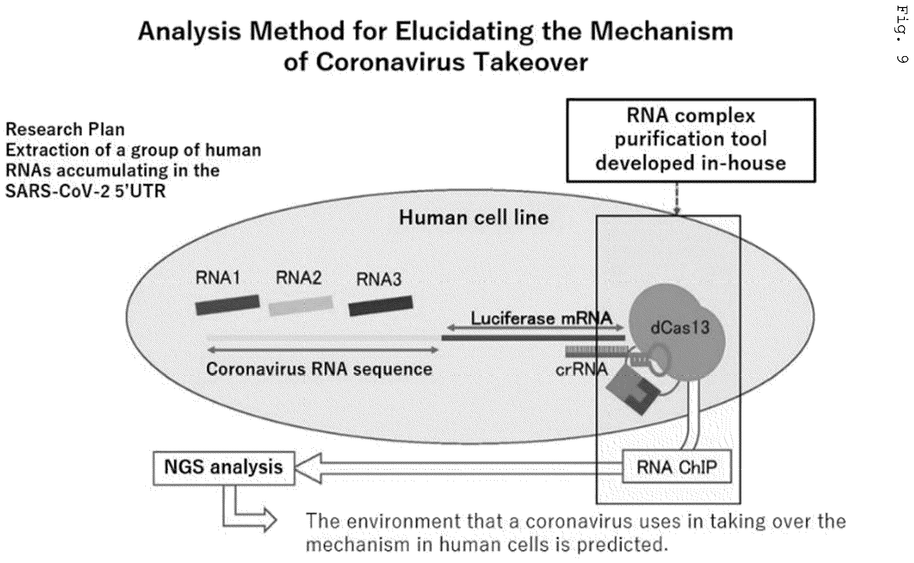

Fig. 8 is a model diagram of performing the TRIP method. Fig. 9 shows an outline of a verification test on the interaction between SARS-CoV-2-derived RNA and human intracellular factors.

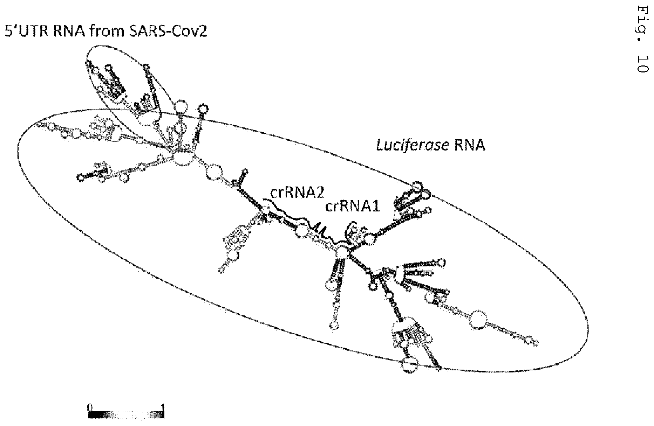

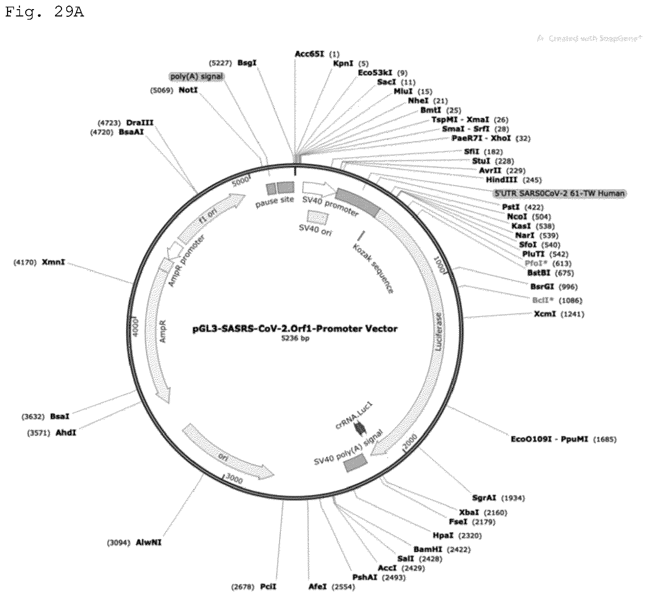

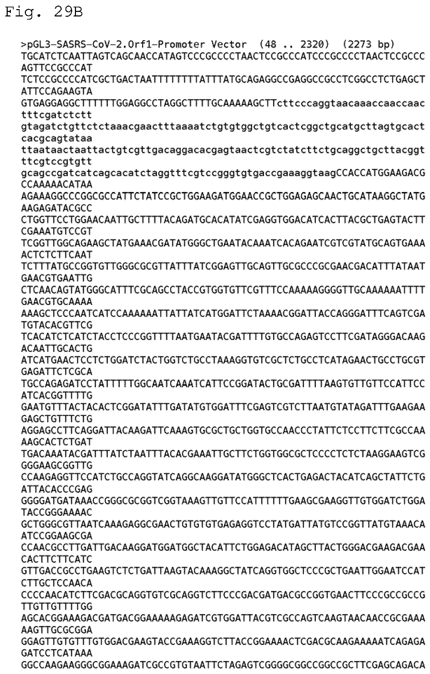

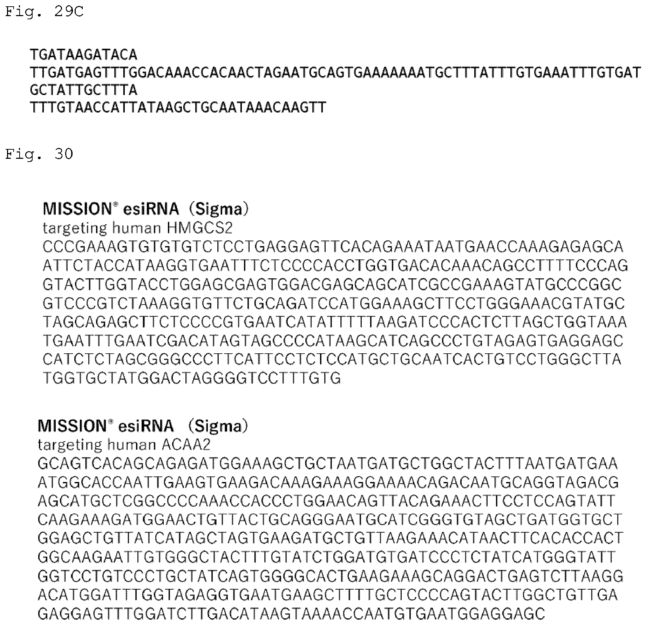

The secondary structure of a virus-derived RNA sequence is predicted and crRNA that can be targeted is designed, or a pGL3-SARS-CoV-2.ORF1-promoter (Fig. 29A) is prepared as a synthetic RNA expression vector fused with virus-derived RNA and a crRNA-targetable Tag sequence (luciferase sequence). In this test, a lentiviral vector that constitutively expresses a crRNA targeting the luciferase gene was created by inserting the XIST gene sequence into pLKO5.U6.crRNA (gene name).tRFP.v1. In a virus-infected cell (or an infected model cell), a crRNA targeting a viral RNA or a crRNA targeting a Tag sequence and dCas 13 are expressed and converted into Activate-Cas13/dCas13 at any time, and then a virus-derived RNA and a complex thereof are purified by using the TRIP method. For DNA/RNA, next-generation sequencing is performed. In the case of proteins, proteome analysis is performed. Fig. 10 shows an outline of the TRIP method actually performed for the untranslated region of SARS-CoV-2. The present inventors attempted to elucidate the mechanism by which the untranslated sequence derived from SARS-CoV-2 synthesizes its own proteins in human cells.

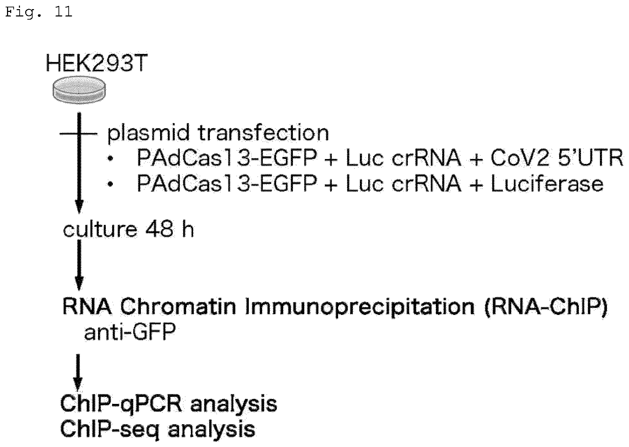

Fig. 11 shows the structural analysis of a fusion RNA of a Tag sequence (in this figure, RNA of the luciferase protein) and the untranslated region of SARS-CoV-2, and a method for designing a crRNA sequence for the TRIP method. The results show that the untranslated region of SARS-CoV-2 has a unique structure.

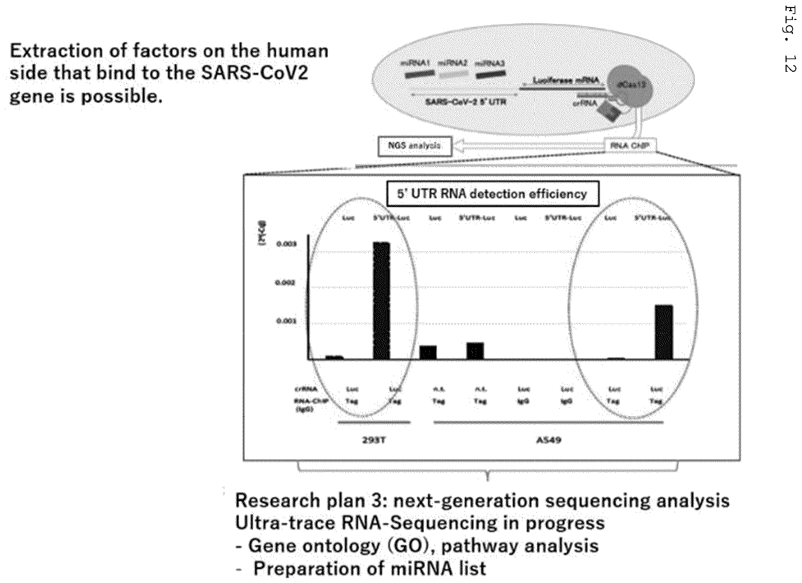

Fig. 12 shows an outline of RNA immunoprecipitation used in the TRIP method.

Fig. 12 shows qPCR analysis for confirming the degree of purity recovered by the TRIP method. After the untranslated region sequence of the Tag-fused SARS-CoV-2 shown in Figs. 9 and 10 or the Tag sequence alone was introduced into HEK293T cells and A549 cells, the TRIP method targeting the Tag sequence was performed to compare and examine the efficiency of recovering the untranslated region sequence of the SARS-CoV-2 by the recovered RNA. Only in cells transfected with Tag-fused SARS-CoV-2 and subjected to Cas13-mediated immunoprecipitation could the RNA sequence be recovered.

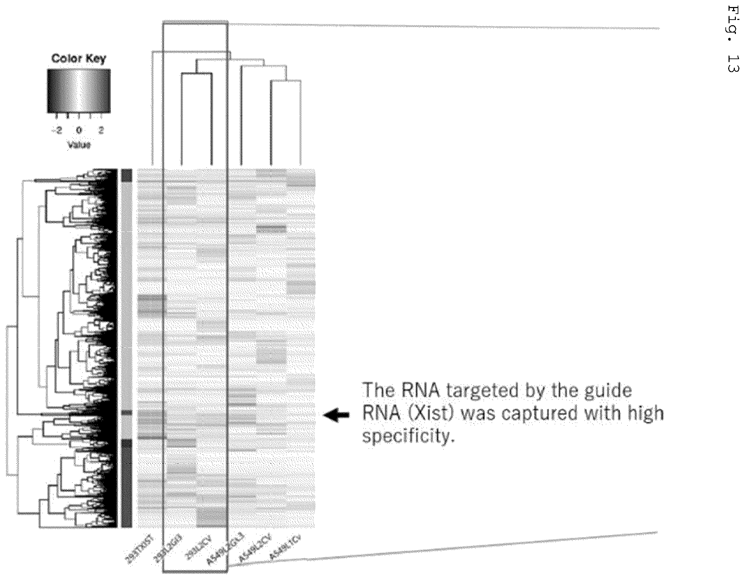

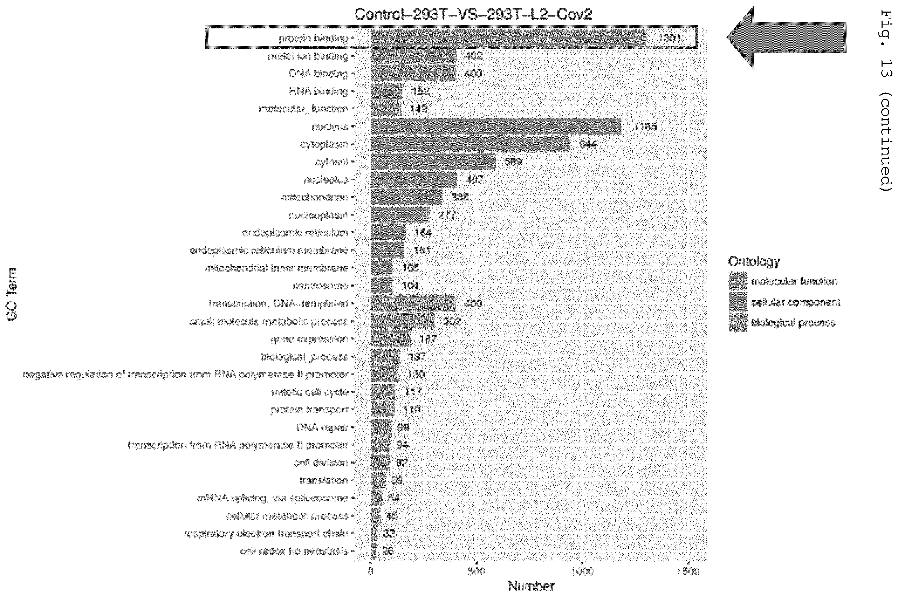

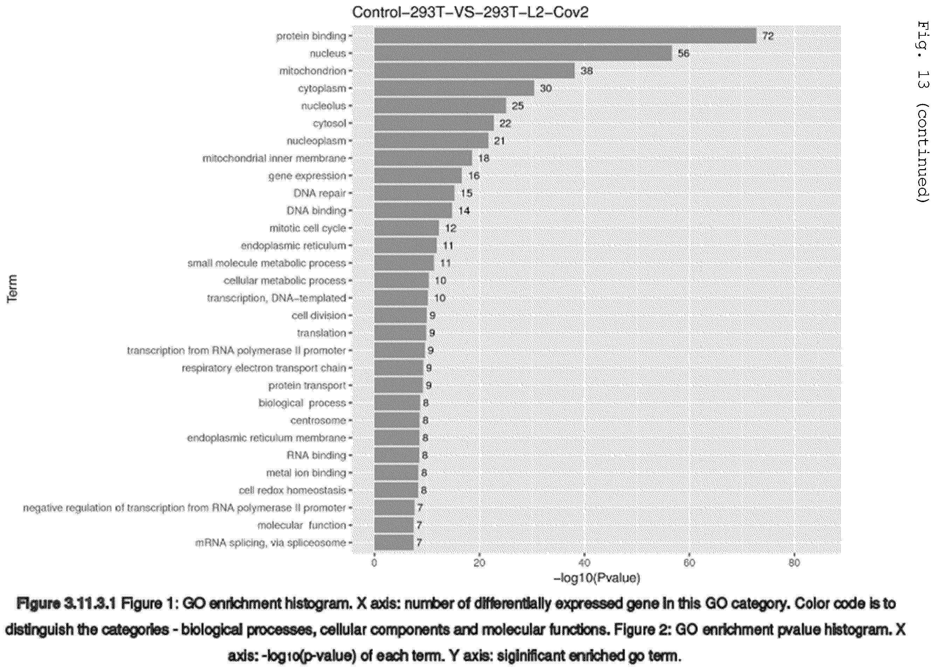

Fig. 13 shows an example of evaluating the performance of the TRIP analysis method targeting SARS-CoV-2. In Fig. 13, the arrow indicates a gene group involved in lipid metabolism obtained by immunoprecipitation. As shown in Fig. 13, it was found that the RNA of the untranslated region sequence of SARS-CoV-2 specifically binds to an RNA sequence in human cells, and significantly binds to a metabolic mechanism in cells.

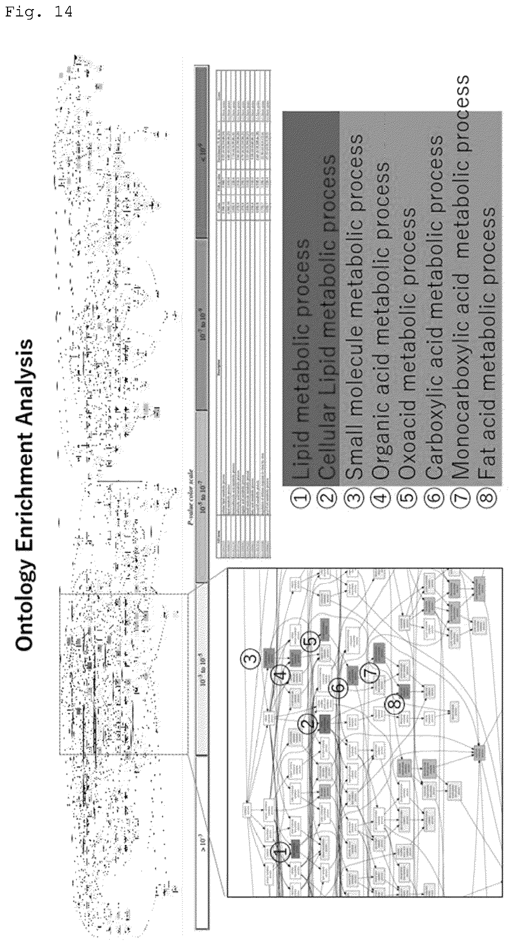

Fig. 14 is another example of evaluating the performance of the TRIP analysis technique targeting SARS-CoV-2. Ontology enrichment analysis revealed that the function of the RNA group recovered in Fig. 13 is a lipid metabolism mechanism.

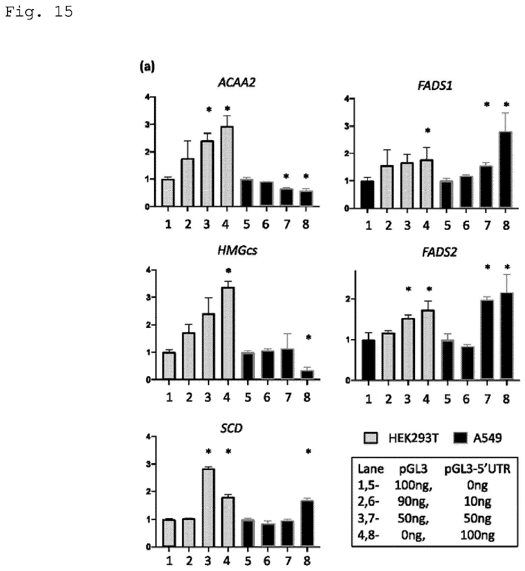

Fig. 15 shows another example of evaluating the performance of the TRIP analysis method targeting SARS-CoV-2: a test for verifying whether a Tag-fused SARS-CoV-2 untranslated region sequence or the Tag sequence alone is introduced into a cell makes a difference in gene expression of the lipid metabolism mechanism in the cell.

Whether the transcriptional activity of genes related to lipid metabolism extracted in the GO analysis is altered by updating the 5' UTR gene expression of SARS-CoV-2 was examined.

ACAA2, HMGcs, FADS 1/2, and SCD are all factors involved in cholesterol metabolism and/or lipid metabolism. When pGL3-5' UTR was expressed in HEK293T and A549 cells, a significant change in gene expression was confirmed compared to the case in which pGL3 was expressed.

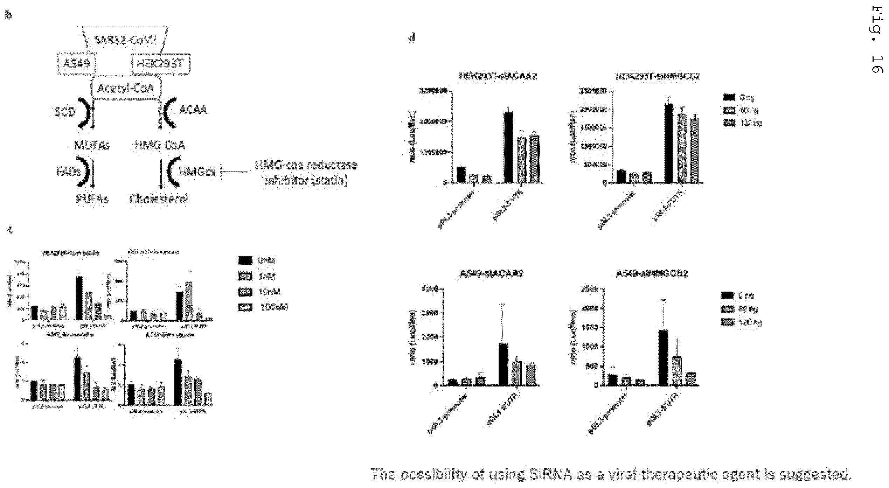

Fig. 16(b) is an interpretation diagram of the results of Fig. 15(a). In the system in which the expression of the 5' UTR derived from SARS-CoV-2 was increased, the metabolism to the PUFA system was enhanced in A549, and the cholesterol metabolism system was enhanced in HEK 293T cells.

Fig. 16(c) shows an attempt to suppress HMGcs using statin, which is a commercially available drug. Statin suppressed the expression of luciferase protein increased by the 5' UTR derived from SARS-CoV-2. Inhibition of cell proliferation and inhibition of transcription of luciferase RNA were not confirmed.

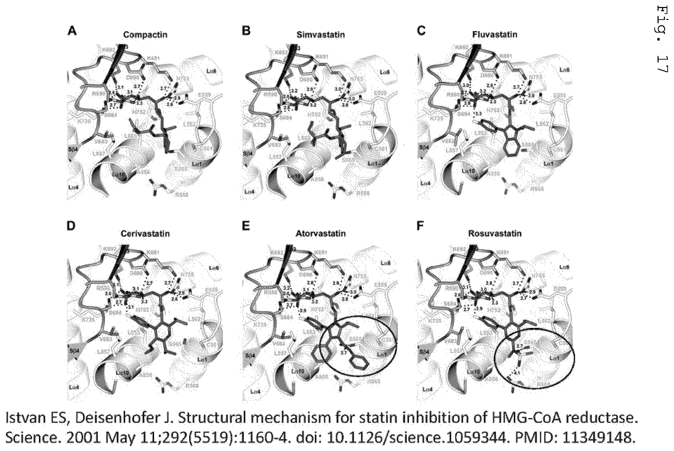

Fig. 16(d) shows that suppression of HMGcs and ACAA2 expression by esiRNAs was performed and the suppression of luciferase protein expression enhanced by the SARS-CoV-2-derived 5' UTR was confirmed. Fig. 17 shows the range of sequences targeted by esiRNA (MISSION (registered trademark) esiRNA: consisting of a heterogeneous pool of several hundred siRNAs of 21bp) .

These results indicate that regulation of lipid metabolism suppresses the expression of luciferase protein, which is enhanced by the 5' UTR derived from SARS-CoV-2. No toxicity to the cell line was confirmed in any of the suppressor systems, nor was suppression of luciferase mRNA expression confirmed. Thus, the suppression of luciferase protein expression enhanced by the SARS-CoV-2-derived 5' UTR is due to translational repression of the luciferase protein.

As shown in Fig. 17, atorvastatin and rosuvastatin have many binding sites (S565) when binding to HMG-CoA, which may be one of the reasons for their inhibitory effects on SARS-CoV-2 growth.

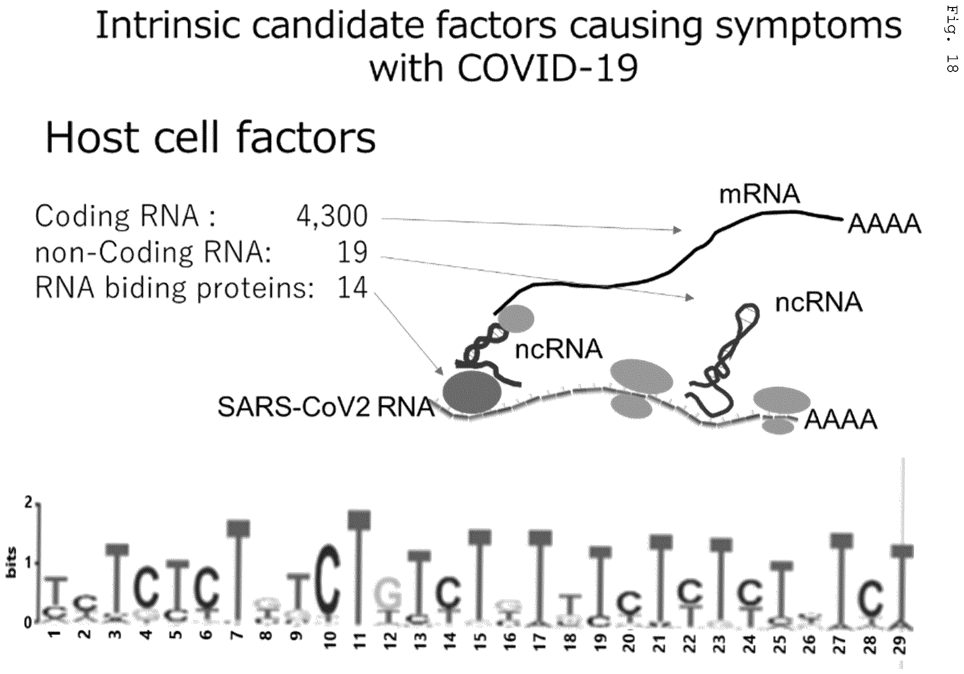

Fig. 18 is another example of evaluating the performance of the TRIP analysis method targeting SARS-CoV-2, showing the law of complex formation by the untranslated region sequence of SARS-CoV-2 in host cells, including human cells. The binding of the untranslated region sequence of SARS-CoV-2 (CoV19 in the figure) and human-derived RNA requires a factor on the human side to have a specific sequence, which predicts the presence of a protein that mediates the binding of the untranslated region sequence of SARS-CoV-2 and human RNA.

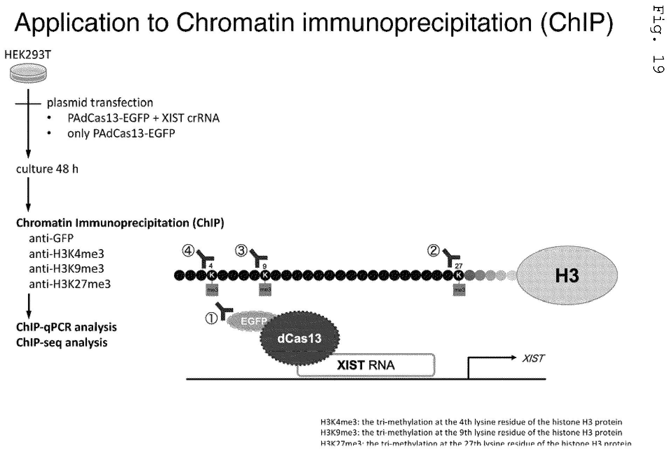

Fig. 19 is an outline of DNA immunoprecipitation used in the TRIP method, showing the procedures for extraction and analysis of DNA bound to XIST RNA.

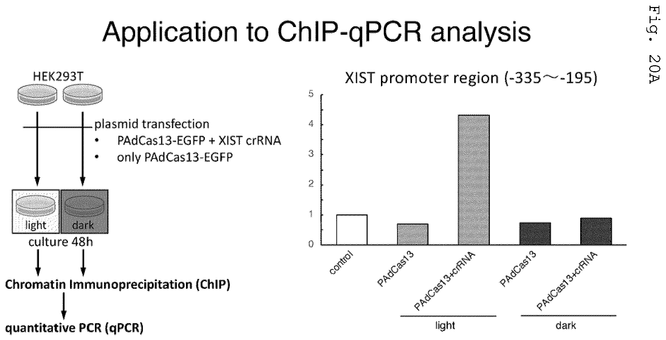

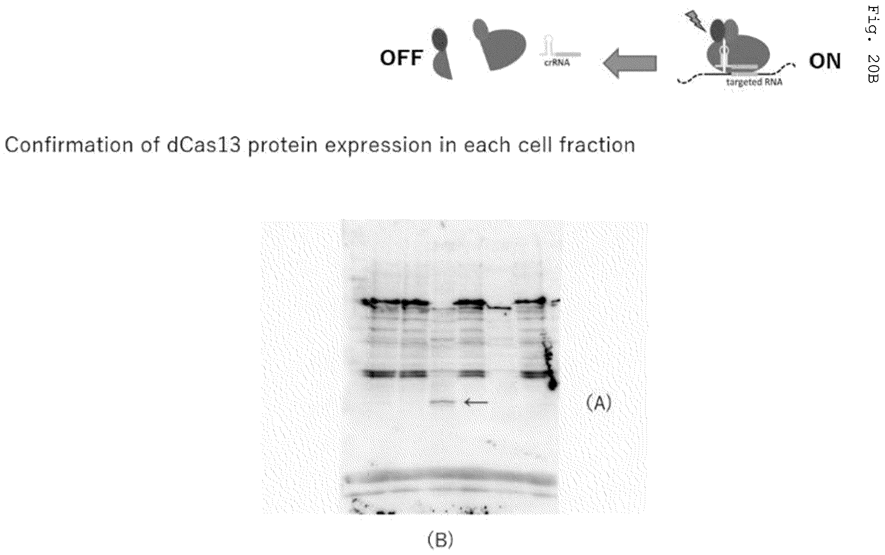

Fig. 20 shows a demonstration test of a photosensitive switching system. Only in light-stimulated cells was it possible to purify the XIST-DNA complex via dCas13-crRNA. The generated genomic DNA was detected by the qPCR method for known XIST RNA binding region.

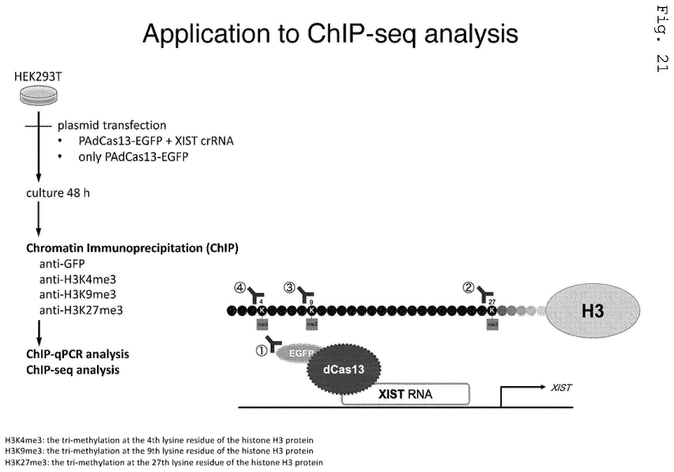

Fig. 21, in addition to Fig. 19, shows an outline of an example of testing whether the binding region of XIST RNA is associated with the histone code.

The diagram on the left in Fig. 22A: a diagram of visualization on whether binding regions on DNA of XIST RNA have regularity around the transcription start region of the gene. The second column from the left shows the test results of the TRIP method performed on XIST. The first column from the left is a negative test without crRNA. The third, fourth, and fifth columns from the left are accumulation diagrams targeting histone modifications. The diagram on the right in Fig. 22A is a graphical representation of the accumulation regions from the transcription start region in the diagram on the left. The top graph shows the test results of the TRIP method performed on XIST. The top graph shows an accumulation pattern similar to that of H3K4me3 in the third graph from the top, which suggests that XIST is a transcriptional regulator.

Fig. 22B shows the results of confirming accumulation into the transcription start region from the results of DNA sequencing of the genomic DNA group targeted by XIST, which are extracted from the results of gRNA-dCas13-mediated pulldown (TRIP) targeting the lncRNA XIST. The isolated DNA is DNA that interacts with XIST RNA and was known to be involved in transcriptional regulation of genes by association with histone modification. The results of this test demonstrate that XIST target sequences could be extracted in a higher accumulation amount (peak height) and with a higher resolution (peak narrowness) than the prior art (e.g., ChIRP; reference: Mol Cell. 2011 Nov 18; 44(4): 667-78).

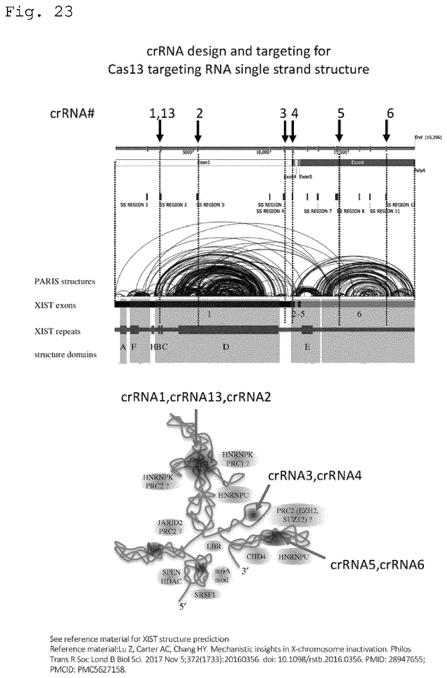

Fig. 23 shows RNA structural analysis data used as a reference in designing XIST crRNA, a structural prediction diagram, and a diagram of a group of proteins predicted to bind to the XIST sequence.

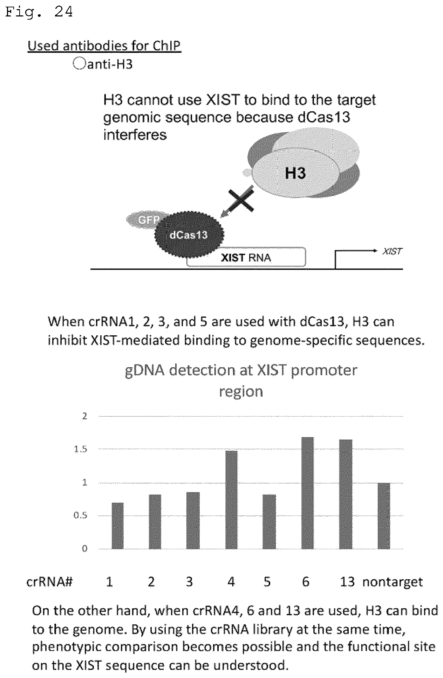

Fig. 24 shows verification test results of XIST RNA function regulation by dCas13. Whether induction of dCas13 to a specific sequence of XIST RNA in cells transfected with crRNA corresponding to the crRNA number indicated in the upper diagram of Fig. 23 would inhibit induction of H3 (histone H3) onto the genome via XIST RNA was confirmed. The results show that binding of H3 to the genomic region is inhibited in cells transfected with crRNAs 1, 2, 3, and 5, thus indicating that XIST function is inhibited when crRNAs 1, 2, 3, and 5 are targeted.

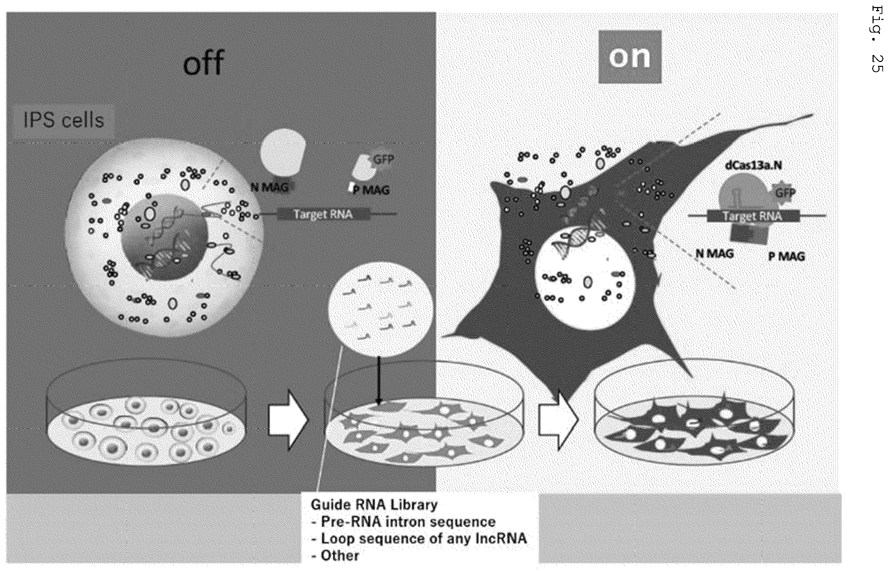

Fig. 25 shows an application using a photoactivated Cas13 (PAdCas13) system. Comprehensive RNA function analysis becomes possible by using the application in Fig. 24 (precision RNA sequence modification (PRSM) method in Fig. 2). As an application example, it becomes possible to inhibit a sequence-specific function of a target RNA at a time desired by a tester in a differentiation induction test using iPS cells.

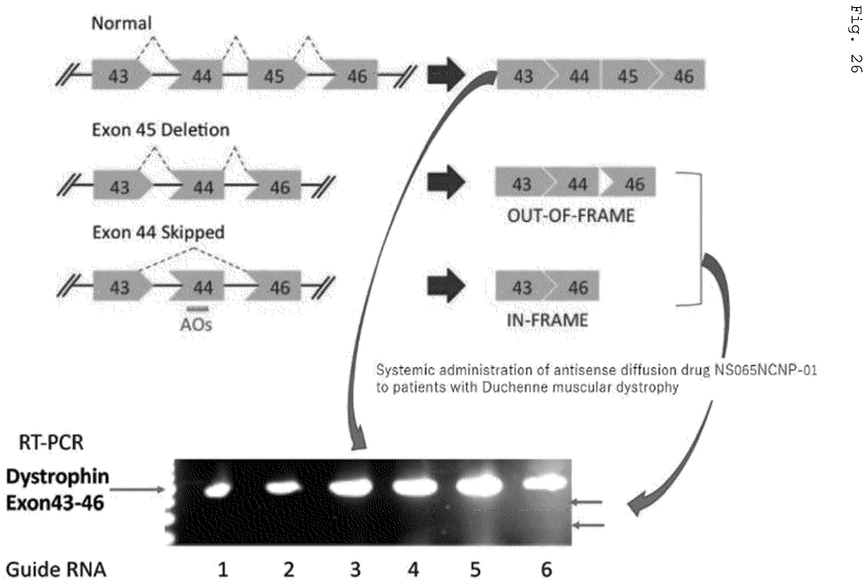

Fig. 26 shows pre-RNA level repair of Duchenne muscular dystrophy causative dystrophin gene mutation. To examine the RNA splicing mechanism regulation by Cas13/dCas13 and the possibility of therapeutic application, splicing regulation of dystrophin gene was attempted. Diseases mainly caused by abnormal RNA splicing are as shown in the accompanying drawings.

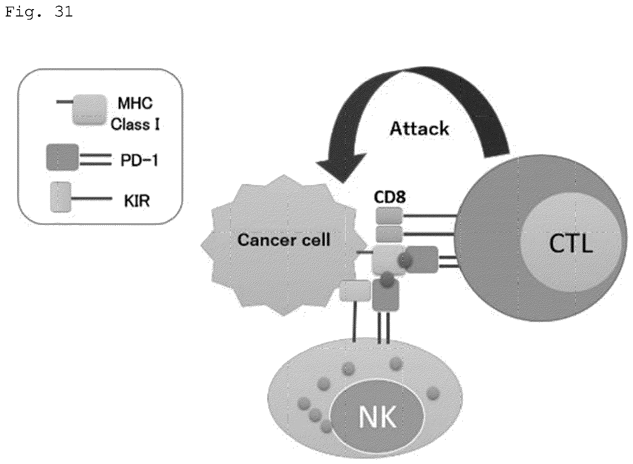

### Example 4: PD-1 Pre-mRNA of PD-1 Positive T Cells/NK Cells (Fig. 31)

T cells, which have the ability to recognize and eliminate cancer cells, express PD-1 and thereby misidentify cancer cells as autologous cells. By using dCas13, genetically unmodified chimeric antigen receptor T cells that intentionally suppress the expression of PD-1 from T cells around cancer cells and specifically attack cancer cells can be created, thus leading to the development of new immunotherapy (Fig. 31).

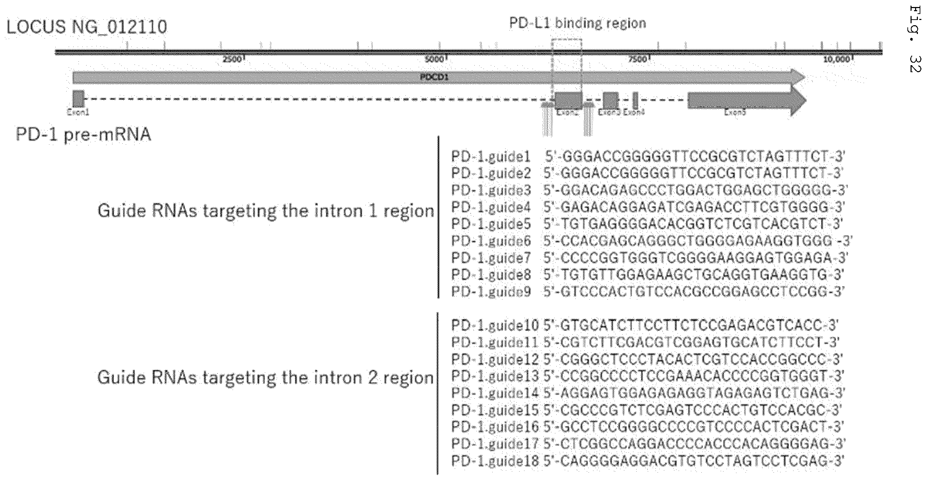

The target was the expression of exon 2 of PD-1 pre-mRNA. The object was to control the progression of PD-1 pre-mRNA to mature mRNA by altering the structure of the functional sequence of the splicing regulation protein that binds to intron 1 and intron 2 of PD-1. The sequences of the guide RNAs targeting the respective sequences are shown (Fig. 32).

### Production of Genetically Unmodified Chimeric Antigen Receptor T Cells with Enhanced Ability to Attack Tumor Cells

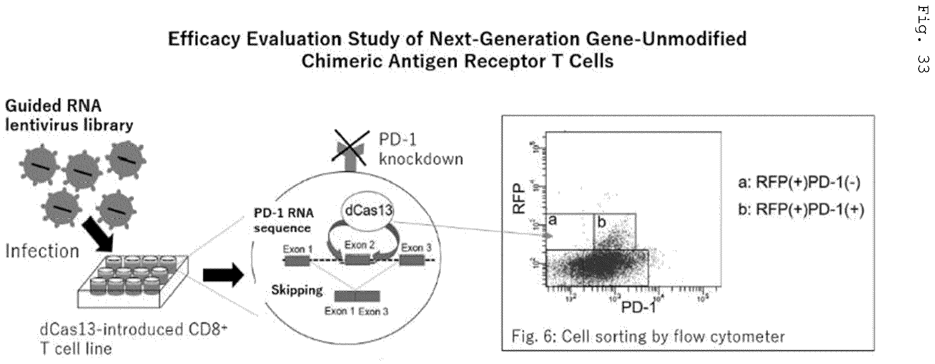

Diagram on the left in Fig. 33: a PA-dCas13 constitutive expression system was introduced into a human CD8⁺ T cell line EBT-8, which is a PD-1 expressing cell, and then guide RNAs targeting PD-1 pre-mRNA were introduced. The guide RNAs were introduced by lentiviral vectors, and PD-1-targeting guide RNAs 1-18 were all introduced at the same time.

Diagram on the right in Fig. 33: selective suppression of PD-1 expression was confirmed in EBT-8 in the area of a.

Chimeric antigen receptor T cells that have succeeded in suppressing the expression are prepared from lymphocytes derived from the ascites fluid of a patient, and the efficacy and safety are evaluated in an animal model of peritoneal dissemination.

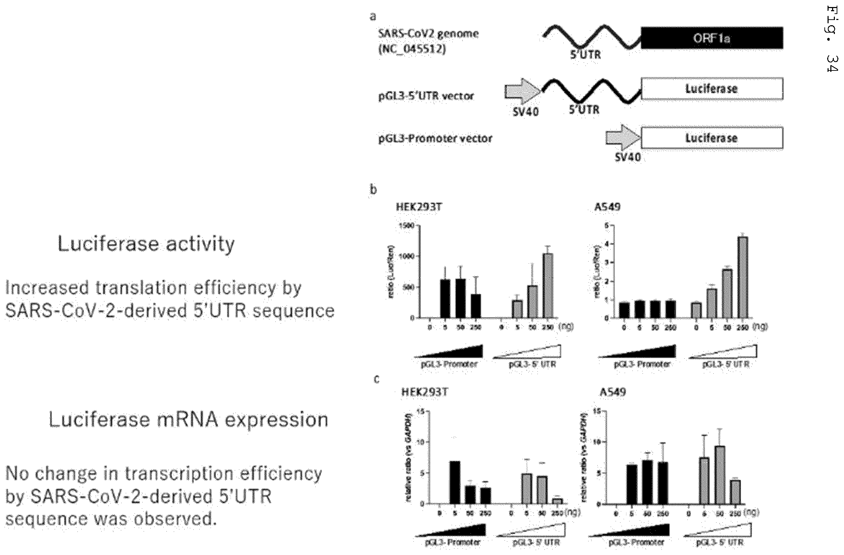

### Example 5: Observation of Increased Translational Activity of Luciferase RNA Fused with the 5' UTR RNA Gene Information Derived from SARS-CoV-2 (Fig. 34)

Fig. 34a: The 5' UTR gene region in the RNA genome of SARS-CoV-2 corresponds to 261nt on the 3' side of the ORF1A sequence. The sequence of the 5' UTR region was inserted in a fused manner to the 3' side of the Kozak sequence of the luciferase RNA sequence in the pGL3-promoter vector.

Fig. 34b: Whether the expression of luciferase protein in HEK 293T and A549 cells increased depending on the amount of pGL3-promoter or pGL3-5' UTR vector introduced in the cells was compared and measured. In HEK293T and A549 cells, increased expression of luciferase protein fused with 5' UTR RNA gene derived from SARS-CoV-2 was observed. This suggests that the 5' UTR derived from SARS-CoV-2 contains sequences that enhance the translational activity of mRNA fused in human cells.

Fig. 34c: Whether the expression level of Luciferase mRNA was changed or not in the test performed in Fig. 34b was confirmed by the qPCR method. No increase in transcription of luciferase mRNA was confirmed.

The above results indicate that the 5' UTR derived from SARS-CoV-2 contains a sequence that enhances the translational activity of mRNA fused in human cells.

The 5' UTR derived from SARS-CoV-2 is expected to be utilized in molecular genetics research as a novel IRES-like sequence that increases the translational efficiency of any mRNA.

## Claims

1. A CRISPR-dCas/Cas protein derivative or CRISPR-dCas/Cas protein derivative set derived from a CRISPR-dCas (dead Cas) protein or a CRISPR-Cas protein,
the CRISPR-dCas (dead Cas) protein having a helix region, being capable of forming a complex with a guide RNA and a target RNA, and having no nuclease activity, and
the CRISPR-Cas protein having a helix region, being capable of forming a complex with a guide RNA and a target RNA, and having a nuclease activity,
the CRISPR-dCas/Cas protein derivative or CRISPR-dCas/Cas protein derivative set comprising
an N domain containing the N-terminal side of the helix region of the CRISPR-dCas (dead Cas) protein or the CRISPR-Cas protein,
a C domain containing the C-terminal side of the helix region of the CRISPR-dCas (dead Cas) protein or the CRISPR-Cas protein, and
first and second factors capable of binding in response to stimulation,
the N domain being linked to the first factor, and the C domain being linked to the second factor,
the CRISPR-dCas/Cas protein derivative having the first and second factors being bound via a linker containing a protease recognition sequence or via a linker containing a self-cleaving peptide, or being non-covalently bound,
the CRISPR-dCas/Cas protein derivative thus having
a structure of (N domain)-(first factor)-(linker containing a protease recognition sequence)-(second factor)-(C domain), or
a structure of (N domain)-(first factor)-(linker containing a self-cleaving peptide)-(second factor)-(C domain), or
a structure of (N domain)-(first factor)-(non-covalent bond)-(second factor)-(C domain), and
the CRISPR-dCas/Cas protein derivative set comprising two portions that are (N domain)-(first factor) and (second factor)-(C domain).

2. The CRISPR-dCas/Cas protein derivative or CRISPR-dCas/Cas protein derivative set according to claim 1, wherein the N domain comprises a part of an amino acid sequence on the N-terminal side of the helix region, and the C domain comprises a part of an amino acid sequence on the C-terminal side of the helix region.

3. The CRISPR-dCas/Cas protein derivative or CRISPR-dCas/Cas protein derivative set according to claim 1, wherein the N domain comprises 164 amino acids in a part of an amino acid sequence on the N-terminal side of the helix region of Cas13a derived from *Leptotrichia wadei* (Sequence ID: WP_21746774.1), and the C domain comprises 1003 amino acids in a part of an amino acid sequence on the C-terminal side of the helix region of Cas13a derived from *Leptotrichia wadei* (Sequence ID: WP_21746774.1) .

4. The CRISPR-dCas/Cas protein derivative or CRISPR-dCas/Cas protein derivative set according to any one of claims 1 to 3, wherein the first factor contains nMAG or pMAG and the second factor contains pMAG or nMAG.

5. A polynucleotide encoding the CRISPR-dCas/Cas protein derivative or CRISPR-dCas/Cas protein derivative set of any one of claims 1 to 4, or a complementary strand thereof.

6. One or two vectors comprising one or two polynucleotides of claim 5 or complementary strands thereof.

7. The one or two vectors according to claim 6, wherein each vector is an adenoviral vector, an adeno-associated viral vector, or a plasmid vector.

8. The one or two vectors according to claim 7, wherein the vectors are two vectors, one vector having a structure of (N domain)-(first factor) and the other vector having a structure of (second factor)-(C domain).

9. The one or two vectors according to any one of claims 6 to 8, further comprising a polynucleotide encoding a guide RNA corresponding to the target RNA.

10. A transformant transformed by the one or two vectors of any one of claims 6 to 9.

11. The CRISPR-dCas/Cas protein derivative or the CRISPR-dCas/Cas protein derivative set according to any of claims 1 to 4, the polynucleotide or complementary strand thereof according to claim 5, the one or two vectors according to any one of claims 6 to 9, or the transformant according to claim 10, wherein the target RNA is a group of long noncoding RNAs (lncRNAs) and/or noncoding RNAs (ncRNAs) that are both associated with biological functions and diseases.

12. The transformant according to claim 10 or 11, wherein the transformant is a transformed mammalian cell or a transformed non-human mammal.

13. The CRISPR-dCas/Cas protein derivative or the CRISPR-dCas/Cas protein derivative set according to any one of claims 1 to 4, the polynucleotide or complementary strand thereof according to claim 5, the one or two vectors according to any one of claims 6 to 9, or the transformant according to any one of claims 10 to 12, wherein the target RNA is an RNA derived from an RNA virus, a novel coronavirus RNA, an RNA derived from a synthetic gene, a lncRNA, an ncRNA of 30 nt or more that can be targeted by a guide RNA, or a pre-RNA.

14. A carrier for purifying a target RNA, comprising
a solid carrier, and
a CRISPR-dCas (dead Cas) protein capable of forming a complex with a guide RNA and a target RNA and having no nuclease activity, the CRISPR-dCas (dead Cas) protein being supported on the solid carrier.

15. A method for purifying a target RNA, comprising:
allowing a guide RNA and a target RNA to act on the carrier of claim 14 to form a complex comprising the target RNA, the guide RNA, and the CRISPR-dCas protein on the carrier; and
eluting the target RNA and a target RNA-binding factor from the complex, the target RNA-binding factor being selected from the group consisting of genomic DNAs, proteins, and RNAs other than the target RNA.

16. A method for analyzing an intracellular environment comprising:
introducing or expressing multiple guide RNAs into the transformant of claim 10;
lysing the transformant to capture a target RNA with RNA capture particles; and
analyzing the target RNA bound to the RNA capture particles.

17. The analysis method according to claim 16, wherein the transformant is an iPS cell.

18. The analysis method according to claim 16 or 17, comprising extracting a phenotype of a cell generated by the formation of a complex of the target RNA, guide RNAs, and a photoactivated dCas protein.

19. A prophylactic or therapeutic agent for a novel coronavirus comprising a lipid metabolism regulator as an active ingredient.

20. The prophylactic or therapeutic agent for a novel coronavirus according to claim 19, wherein the lipid metabolism regulator has an HMG-CoA reductase inhibitory action or an acetyl-CoA reductase inhibitory action.

21. The prophylactic or therapeutic agent for a novel coronavirus according to claim 19 or 20, wherein the lipid metabolism regulator is atorvastatin or rosuvastatin.

22. A therapeutic agent for diseases based on abnormal splicing of mRNA, rRNA (ribosomal RNA), and lncRNA, the therapeutic agent comprising the one or two vectors of any one of claims 6 to 9 as an active ingredient.

23. The CRISPR-dCas/Cas protein derivative or the CRISPR-dCas/Cas protein derivative set according to claim 1, wherein the CRISPR-dCas/Cas protein is Cas13 protein having nuclease activity.
